# EUROPEAN PATENT APPLICATION

(11) **EP 4 821 949 A1**
(43) Date of publication of application: **14.10.2026**
(21) Application number: 24899963.3
(22) Date of filing: 06.12.2024
(51) Int. Cl.: C07K 16/44, C07K 16/46, C12N 15/13, C12N 15/63, C12N 5/10, G01N 33/53, A61K 39/395

(54) **MOLECULE BINDING TO AMINO ACID LINKER AND USE THEREOF**

(30) Priority: 08.12.2023 CN 202311692347
(71) Applicant: Nanjing GenScript Biotech Co., Ltd., Nanjing, Jiangsu 211100 (CN)
(72) Inventor: QIN, Xijian, Nanjing, Jiangsu 211100 (CN); WANG, Fei, Nanjing, Jiangsu 211100 (CN)
(74) Representative: Synergy IP Group AG
(86) International application number: PCT/CN2024/137418
(87) International publication number: WO 2025/119330

(57) **Abstract**

The present application relates to a molecule specifically binding to the amino acid linker, and the use of the antibody in the detection of the amino acid linker or in the construction of a bispecific antibody and a universal CAR-T composition.

## Description

### CROSS-REFERENCE TO RELATED APPLICATIONS

The present application claims priority to Chinese Patent Application CN 202311692347.2, filed on December 8, 2023, which is incorporated herein by reference in its entirety.

### TECHNICAL FIELD

The present application relates to a molecule specifically binding to an amino acid linker, and the use of the molecule in the detection of the amino acid linker or in the construction of a bispecific antibody and a universal CAR-T composition.

### BACKGROUND

In recombinant biomacromolecules, linkers are often used to link two or more functional modules, forming an integrated structure. For example, linkers can be used to link proteins, nucleic acids, and/or polypeptides, enabling specific functional combinations or enhancements. In some cases, linkers also serve to separate two or more functional modules, allowing various modules to perform their respective functions without interfering with each other.

The selection of linkers in recombinant biomacromolecules mainly depends on the types of molecules to be linked, the required stability of the linkage, and the specific requirements of subsequent applications. Amino acid linkers, e.g., glycine-serine linkers (i.e., GS linkers), are among the most commonly used linkers. They can be designed into various sequence combinations and lengths in the form of (GnS)m, where n and m may be integers of at least 1. (G₂S)₂, (G₂S)₄, (G₃S)₃, (G₄S)₁, (G₄S)₂, and (G₄S)₃ are several commonly used GS linkers, which are often used to link different functional domains of proteins or polypeptides, and have characteristics of flexibility and extensibility. These linker sequences are widely used in many research fields, e.g., protein engineering, drug delivery systems, etc.

GS linkers are often used to link the heavy chain variable region (VH) and the light chain variable region (VL) in a single-chain variable fragment (scFv). Compared with classical IgG antibody molecules, scFv has the advantages including small molecular weight, strong tissue penetration, low immunogenicity, and high specificity, and therefore has attracted increasing attention in applications such as targeted therapy, imaging diagnosis, and cell therapy. In the field of targeted therapy, drugs or toxins can be conjugated to scFv. By virtue of the specificity and low immunogenicity of scFv, drugs are delivered to target tissues, thereby achieving specific killing of target cells. In imaging diagnosis, due to its strong tissue penetration and short half-life, scFv exhibits a higher distribution index in tumour tissues compared to full-length antibodies during radiological imaging, while posing lower risks to the body. In cell therapy, scFv serves as the extracellular domain of CAR in chimeric antigen receptor T cells (CAR-T), determining the targeting specificity and therapeutic efficacy of CAR-T cells. In biological monitoring, due to its high affinity and ease of preparation, scFv is often used for detecting harmful substances in water sources, food, etc. Furthermore, due to its simple structure, scFv is often used in the construction of bispecific antibodies.

GS linkers are also often used to link two or more nanobodies to prolong half-life and enhance single-target recognition specificity, or to construct bispecific or multispecific antibodies targeting two or more antigens, thereby enabling better targeting of certain cells in specific environments, such as tumour cells in the tumour microenvironment. CARs may also contain two or more nanobodies linked via GS linkers in the extracellular domains to reduce the risk of off-target effects.

Many recombinant proteins containing GS linkers, such as the above scFv, or CARs or multispecific antibodies containing two or more nanobodies, lack an Fc region. Therefore, the most commonly used universal Fc-targeting antibodies cannot be used to detect or quantify these recombinant antibodies. Currently, the detection of scFv-containing proteins generally relies on protein L, anti-Fab antibodies, etc., but all have certain limitations. For example, protein L mainly targets the κ subtype of light chains and fails to detect other subtypes. In addition, protein L suffers from problems such as high non-specific binding and low detection rates. Anti-Fab antibodies are mostly polyclonal, which leads to significant batch-to-batch variation, as well as problems such as low universality and poor specificity. Therefore, alternative methods with high specificity and good universality are needed for the detection of scFv antibodies, etc.

### SUMMARY

The inventors of the present application have prepared and screened antibodies that specifically bind to GS linkers. These antibodies enable localization, quantification, and signal amplification of GS linker-containing proteins by specifically binding to GS linkers on proteins, e.g., scFvs and chimeric antigen receptors (CARs), thereby detecting the expression, metabolism, distribution, etc., of GS linker-containing proteins. Compared with conventional methods using protein L, anti-Fab antibodies, target antigens, anti-idiotypic antibodies, etc., the antibodies of the present application: i) have a certain degree of universality, i.e., are applicable to most GS linker-containing proteins regardless of the target of the protein, the subtype of the Fc region contained in the protein, the subtype of the antibody light chain, the antibody form (such as single-domain antibody or scFv), etc., and are applicable to most GS linkers other than (G₄S)₁; ii) have high binding specificity for GS linker-containing proteins, with higher detection accuracy than the protein L assay, and stronger population resolution ability for CAR-T cells based on GS linker-containing scFvs than anti-Fab antibodies; and iii) have high binding sensitivity to GS linker-containing proteins, enabling the detection of 2 pmol of a GS linker-containing protein.

In addition, the GS antibody of the present application can also be used for the construction of universal CAR-T cells. Specifically, the GS antibody of the present application or an antigen-binding portion thereof, e.g., in the form of an scFv (referred to as anti-GS-CAR), can be contained in the extracellular domain of a CAR, and an antibody or an antigen-binding portion thereof that contains a GS linker and that targets a disease-associated antigen (referred to as a disease-targeting antibody) can be constructed. In this way, T cells expressing the anti-GS-CAR can recognize cells expressing the disease-associated antigen via the disease-targeting antibody, and elicit an attack on such cells. Alternatively, a GS linker may be contained in the extracellular domain of a CAR (referred to as GS linker-CAR), and a bispecific molecule comprising the GS antibody of the present application or an antigen-binding portion thereof as well as an antibody or an antigen-binding portion thereof targeting a disease-associated antigen (referred to as a GS linker + disease dual-targeting antibody) may be constructed. In this way, T cells expressing the GS linker-CAR can recognize cells expressing the disease-associated antigen via the GS linker + disease dual-targeting antibody, and elicit an attack against such cells. Construction of the above universal CAR-T cells in combination with the corresponding disease-targeting antibodies or GS linker + disease dual-targeting antibodies can reduce the cost of cell therapy to a certain extent.

In addition, the GS antibody of the present application can also be used for the purification of GS linker-containing proteins. Specifically, the antibody of the present disclosure can be conjugated to a solid support. The solid support includes magnetic beads, resin, agarose beads, etc. A prokaryotic or eukaryotic lysate or cell culture supernatant containing a GS linker-containing protein is contacted with a GS antibody-containing solid support. The GS linker-containing protein is then specifically captured by the GS antibody. Subsequently, the impurity proteins that do not bind to the GS antibody are washed away. Finally, the GS linker-containing protein is separated from the GS antibody by altering conditions such as pH or salt concentration, yielding the GS linker-containing protein with high purity. This method provides a novel purification strategy for GS linker-containing proteins and simplifies the purification process for such proteins.

Therefore, in a first aspect, the present application provides an isolated monoclonal antibody or an antigen-binding portion thereof that is capable of specifically binding to a GS linker. The antibody or the antigen-binding portion thereof of the present application may be, e.g., rabbit-derived, chimeric, or humanized.

The antibody or the antigen-binding portion thereof of the present application may comprise: i) a heavy chain variable region, which may comprise VH CDR1, VH CDR2, and VH CDR3, wherein VH CDR1, VH CDR2, and VH CDR3 may comprise amino acid sequences set forth in: (1) NYAIM (SEQ ID NO: 1), VIYANGDPYCASWAKG (SEQ ID NO: 5), and GGF, respectively; (2) NYDMY (SEQ ID NO: 2), VSYKNGRAHYASWAKG (SEQ ID NO: 8), and GPL, respectively; (3) NYDMY (SEQ ID NO: 2), VSYASGRTYSVRWAKG (SEQ ID NO: 10), and GPL, respectively; (4) NYDMY (SEQ ID NO: 2), VIYKNGNAHSASWARG (SEQ ID NO: 12), and GPF, respectively; (5) NHAIM (SEQ ID NO: 3), VIYSNGNPYCARWVKG (SEQ ID NO: 7), and GGF, respectively; (6) NYDMY (SEQ ID NO: 2), VIYVNGNTHYASWAKG (SEQ ID NO: 6), and GPF, respectively; (7) NYDMY (SEQ ID NO: 2), CIYTGSGGKRYANWAKG (SEQ ID NO: 9), and GPF, respectively; or (8) TNAMT (SEQ ID NO: 4), TITISGNKYYASWAKG (SEQ ID NO: 11), and GVVQSLVL (SEQ ID NO: 13), respectively; or ii) a light chain variable region, which may comprise VL CDR1, VL CDR2, and VL CDR3, wherein VL CDR1, VL CDR2, and VL CDR3 may comprise amino acid sequences set forth in: (1) QSSQSIYNKNDLV (SEQ ID NO: 14), GISTLDS (SEQ ID NO: 20), and LGGFSCSSGDCGA (SEQ ID NO: 25), respectively; (2) QASQSVWNNNDLV (SEQ ID NO: 17), DASTLSS (SEQ ID NO: 21), and LGGFSCSTGDCGA (SEQ ID NO: 28), respectively; (3) QASQTVWKNNDLV (SEQ ID NO: 18), DASTLSS (SEQ ID NO: 21), and LGGFSCSSGDCGA (SEQ ID NO: 25), respectively; (4) QASQSVWNNNDLV (SEQ ID NO: 17), DASTLSS (SEQ ID NO: 21), and LGGFSCSRGDCGS (SEQ ID NO: 31), respectively; (5) KTSQSIYNNNDLV (SEQ ID NO: 16), GVSTLDS (SEQ ID NO: 22), and LGGFRCSSGDCGA (SEQ ID NO: 27), respectively; (6) QASQSVWKNKDLV (SEQ ID NO: 15), DASTLSS (SEQ ID NO: 21), and LGGFSCSRGDCGA (SEQ ID NO: 26), respectively; (7) QASQSVWNNNDLV (SEQ ID NO: 17), DASILSS (SEQ ID NO: 23), and LGGFSCRSGDCGA (SEQ ID NO: 29), respectively; or (8) RSSQNVYNNNGLG (SEQ ID NO: 19), DAADLAS (SEQ ID NO: 24), and AGGYSSGSIDNT (SEQ ID NO: 30), respectively. Further provided is a variant of the antibody or the antigen-binding portion thereof described above, which comprises up to about 3 amino acid residue substitutions, e.g., 1, 2, or 3 amino acid residue substitutions, in each CDR, as compared to the antibody or the antigen-binding portion thereof described above.

The isolated monoclonal antibody or the antigen-binding portion thereof of the present application may comprise a heavy chain variable region and a light chain variable region, wherein VH CDR1, VH CDR2, VH CDR3, VL CDR1, VL CDR2, and VL CDR3 may comprise amino acid sequences set forth in: (1) NYAIM (SEQ ID NO: 1), VIYANGDPYCASWAKG (SEQ ID NO: 5), GGF, QSSQSIYNKNDLV (SEQ ID NO: 14), GISTLDS (SEQ ID NO: 20), and LGGFSCSSGDCGA (SEQ ID NO: 25), respectively; (2) NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively; (3) NYDMY (SEQ ID NO: 2), VSYASGRTYSVRWAKG (SEQ ID NO: 10), GPL, QASQTVWKNNDLV (SEQ ID NO: 18), DASTLSS (SEQ ID NO: 21), and LGGFSCSSGDCGA (SEQ ID NO: 25), respectively; (4) NYDMY (SEQ ID NO: 2), VIYKNGNAHSASWARG (SEQ ID NO: 12), GPF, QASQSVWNNNDLV (SEQ ID NO: 17), DASTLSS (SEQ ID NO: 21), and LGGFSCSRGDCGS (SEQ ID NO: 31), respectively; (5) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively; (6) NYDMY (SEQ ID NO: 2), VIYVNGNTHYASWAKG (SEQ ID NO: 6), GPF, QASQSVWKNKDLV (SEQ ID NO: 15), DASTLSS (SEQ ID NO: 21), and LGGFSCSRGDCGA (SEQ ID NO: 26), respectively; (7) NYDMY (SEQ ID NO: 2), CIYTGSGGKRYANWAKG (SEQ ID NO: 9), GPF, QASQSVWNNNDLV (SEQ ID NO: 17), DASILSS (SEQ ID NO: 23), and LGGFSCRSGDCGA (SEQ ID NO: 29), respectively; or (8) TNAMT (SEQ ID NO: 4), TITISGNKYYASWAKG (SEQ ID NO: 11), GVVQSLVL (SEQ ID NO: 13), RSSQNVYNNNGLG (SEQ ID NO: 19), DAADLAS (SEQ ID NO: 24), and AGGYSSGSIDNT (SEQ ID NO: 30), respectively. Further provided is a variant of the antibody or the antigen-binding portion thereof described above, which comprises up to about 3 amino acid residue substitutions, e.g., 1, 2, or 3 amino acid residue substitutions, in each CDR, as compared to the antibody or the antigen-binding portion thereof described above.

The heavy chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 32, 35, 37, 39, 34, 33, 36, or 38. The amino acid sequences of SEQ ID NOs: 32, 35, 37, 39, 34, 33, 36, and 38 may be encoded by nucleotide sequences set forth in SEQ ID NOs: 48, 51, 53, 55, 50, 49, 53, and 54, respectively.

The light chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 40, 43, 45, 47, 42, 41, 44, or 46. The amino acid sequences of SEQ ID NOs: 40, 43, 45, 47, 42, 41, 44, and 46 may be encoded by a nucleotide sequence set forth in SEQ ID NO: 56, 59, 61, 63, 58, 57, 60, and 62, respectively.

The antibody or the antigen-binding portion thereof of the present application may comprise a heavy chain variable region and a light chain variable region, wherein the heavy chain variable region and the light chain variable region may comprise amino acid sequences having at least 80%, 85%, 90%, 91%, 92%, 93%, 94%, 95%, 96%, 97%, 98%, 99% or 100% sequence identity to: (1) SEQ ID NOs: 32 and 40, respectively; (2) SEQ ID NOs: 35 and 43, respectively; (3) SEQ ID NOs: 37 and 45, respectively; (4) SEQ ID NOs: 39 and 47, respectively; (5) SEQ ID NOs: 34 and 42, respectively; (6) SEQ ID NOs: 33 and 41, respectively; (7) SEQ ID NOs: 36 and 44, respectively; or (8) SEQ ID NOs: 38 and 46, respectively.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2, and VL CDR3 may comprise amino acid sequences set forth in NYAIM (SEQ ID NO: 1), VIYANGDPYCASWAKG (SEQ ID NO: 5), GGF, QSSQSIYNKNDLV (SEQ ID NO: 14), GISTLDS (SEQ ID NO: 20), and LGGFSCSSGDCGA (SEQ ID NO: 25), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 32. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 40.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2, and VL CDR3 may comprise amino acid sequences set forth in NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 35. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 43.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2, and VL CDR3 may comprise amino acid sequences set forth in NYDMY (SEQ ID NO: 2), VSYASGRTYSVRWAKG (SEQ ID NO: 10), GPL, QASQTVWKNNDLV (SEQ ID NO: 18), DASTLSS (SEQ ID NO: 21), and LGGFSCSSGDCGA (SEQ ID NO: 25), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 37. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 45.

In some embodiments, the isolated monoclonal antibody or the antigen-binding portion thereof of the present application may comprise a heavy chain variable region and a light chain variable region, wherein VH CDR1, VH CDR2, VH CDR3, VL CDR1, VL CDR2, and VL CDR3 may comprise amino acid sequences set forth in NYDMY (SEQ ID NO: 2), VIYKNGNAHSASWARG (SEQ ID NO: 12), GPF, QASQSVWNNNDLV (SEQ ID NO: 17), DASTLSS (SEQ ID NO: 21), and LGGFSCSRGDCGS (SEQ ID NO: 31), respectively. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 39. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 47.

The isolated monoclonal antibody or the antigen-binding portion thereof of the present application may comprise a heavy chain constant region and/or a light chain constant region, wherein the N-terminus of the heavy chain constant region is linked to the C-terminus of the heavy chain variable region, and the N-terminus of the light chain constant region is linked to the C-terminus of the light chain variable region. The heavy chain constant region may be an IgG, IgD, IgA, IgM, or IgE heavy chain constant region, or a functional fragment thereof, e.g., a fragment comprising the hinge region, CH₂, and CH₃ of the heavy chain constant region. In one embodiment, the heavy chain constant region may comprise an amino acid sequence set forth in SEQ ID NO: 64. The light chain constant region may be a κ or λ light chain constant region. In some embodiments, the light chain constant region may comprise an amino acid sequence set forth in SEQ ID NO: 66. The amino acid sequences set forth in SEQ ID NO: 64 and SEQ ID NO: 66 may be encoded by nucleotide sequences set forth in SEQ ID NO: 65 and SEQ ID NO: 67, respectively.

In some embodiments, the antibody or the antigen-binding antigen of the present application comprises or consists of two heavy chains and two light chains, wherein each of the heavy chains comprises the heavy chain constant region sequence, heavy chain variable region sequence, and/or CDR sequences described above, and each of the light chains comprises the light chain constant region sequence, light chain variable region sequence, and/or CDR sequences described above. In some embodiments, the antibody or the antigen-binding portion thereof of the present application may be a Fab, an F(ab')₂ fragment, Fv, scFv, (scFv)₂, etc.

In some embodiments, the antibody or the antigen-binding portion thereof of the present application may be a full-length antibody. In some embodiments, the antibody or the antigen-binding portion thereof of the present application may be a single-chain antibody (scFv).

The antibody or the antigen-binding portion thereof of the present application may comprise a detectable substance. For example, the antibody or the antigen-binding portion thereof may be fused (e.g., by recombinant expression) with or conjugated to a detectable substance. The antibody or the antigen-binding portion thereof of the present application may comprise a detectable substance at any position that does not affect the antigen-binding property of the antibody or the antigen-binding portion thereof, e.g., in a heavy chain constant region, a light chain constant region, a framework (FR) region of the heavy chain variable region, or a framework region of the light chain variable region. In particular, the antibody or the antigen-binding portion thereof may comprise a detectable substance in a framework region of a heavy chain constant region or a light chain constant region. The detectable substance may be a fluorescent label or a chemiluminescent label. The fluorescent label may be fluorescein isothiocyanate (FITC), phycoerythrin (PE), allophycocyanin (APC), or other dyes that can emit fluorescence upon excitation with light, such as iFluor, Alexa fluor, and Bright violet dyes. The chemiluminescent label may be peroxidase (e.g., horseradish peroxidase (HRP)), alkaline phosphatase (AP), etc.

The present application further provides a bispecific molecule, which comprises the antibody or the antigen-binding portion thereof of the present application that specifically binds to a GS linker. For example, the bispecific molecule of the present application may comprise: i) the antibody or the antigen-binding portion thereof of the present application that specifically binds to a GS linker, and ii) an antibody or an antigen-binding portion thereof that targets a disease-associated antigen. i) and ii) may be linked together, for example, fused together by recombinant expression, or conjugated together via, e.g., a disulfide bond, an amino group, etc. The disease-associated antigen may be, for example, a tumour-associated antigen. In particular, in the bispecific molecule of the present application, the antibody or the antigen-binding portion thereof that specifically binds to a GS linker may be humanized. In particular, in the bispecific molecule of the present application, the antibody or the antigen-binding portion thereof that targets a disease-associated antigen may be human-derived or humanized.

The present application further provides a chimeric antigen receptor (CAR), which may comprise: a) an extracellular domain, which may comprise the antibody or the antigen-binding portion thereof of the present application that specifically binds to a GS linker, particularly the antibody of the present application in the form of an scFv; b) a transmembrane domain; and c) an intracellular signalling domain. The present application further provides an immune cell comprising the chimeric antigen receptor described above, e.g., a T cell. In particular, in the chimeric antigen receptor of the present application, the antibody or the antigen-binding portion thereof that specifically binds to a GS linker may be humanized.

The present application further encompasses a nucleic acid molecule encoding the antibody or the antigen-binding portion thereof, the bispecific molecule, or the chimeric antigen receptor of the present application. The nucleic acid molecule of the present application may comprise a nucleotide sequence set forth in any one of SEQ ID NOs: 48-63. The present application further provides an expression vector and a host cell. The expression vector may comprise the nucleic acid molecule of the present application. The host cell may comprise the expression vector of the present application, or have the nucleic acid molecule of the present application integrated into the genome thereof.

The present application further provides a method for preparing the antibody or the antigen-binding portion thereof, the bispecific molecule, or the chimeric antigen receptor of the present application using the host cell of the present application, the method comprising: (i) expressing the antibody or the antigen-binding portion thereof, the bispecific molecule, or the chimeric antigen receptor in the host cell, and (ii) isolating the antibody or the antigen-binding portion thereof, the bispecific molecule, or the chimeric antigen receptor from the host cell or a culture thereof. In some embodiments, the method may further comprise conjugating a detectable substance to the antibody or the antigen-binding portion thereof of the present application. In particular, the method may further comprise contacting the detectable substance with the antibody or the antigen-binding portion thereof of the present application such that the detectable substance is conjugated to the antibody or the antigen-binding portion thereof of the present application.

The present application further provides a composition comprising the antibody or the antigen-binding portion thereof, the bispecific molecule, the chimeric antigen receptor, the nucleic acid molecule, the expression vector, or the host cell of the present application.

In some embodiments, the antibody or the antigen-binding portion thereof of the present application may comprise a detectable substance, e.g., a fluorescent label or a chemiluminescent label. For example, the antibody or the antigen-binding portion thereof may be fused (e.g., by recombinant expression) with or conjugated to a detectable substance. The antibody or the antigen-binding portion thereof of the present application may comprise a detectable substance at any position that does not affect the antigen-binding property of the antibody or the antigen-binding portion thereof, e.g., in a heavy chain constant region, a light chain constant region, a framework region of the heavy chain variable region, or a framework region of the light chain variable region. In particular, the antibody or the antigen-binding portion thereof may comprise a detectable substance in a framework region of a heavy chain constant region or a light chain constant region.

In some embodiments, the antibody or the antigen-binding portion thereof of the present application may comprise a chemiluminescent label, and the composition may further comprise a reagent that causes the chemiluminescent label to emit light. In some embodiments, the chemiluminescent label may be horseradish peroxidase, and the reagent that causes the chemiluminescent label to emit light may be a peroxide (e.g., H₂O₂) and 3,3'-diaminobenzidine (DAB), or a peroxide (e.g., H₂O₂) and 3,3',5,5'-tetramethylbenzidine (TMB). In some embodiments, the chemiluminescent label may be alkaline phosphatase, and the reagent that causes the chemiluminescent label to emit light may be 4-nitrophenyl phosphate disodium salt hexahydrate (pNPP).

In some embodiments, the antibody or the antigen-binding portion thereof of the present application comprises a heavy chain constant region (e.g., an Fc region), and the composition further comprises an antibody that specifically binds to the heavy chain constant region (e.g., the Fc region) and comprises a detectable substance. In some embodiments, the antibody or the antigen-binding portion thereof of the present application comprises a heavy chain constant region (e.g., an Fc region), and the composition further comprises an antibody that specifically binds to the heavy chain constant region (e.g., the Fc region) and comprises a fluorescent label. The fluorescent label may be fluorescein isothiocyanate. In some embodiments, the antibody or the antigen-binding portion thereof of the present application comprises a heavy chain constant region (e.g., an Fc region), the composition further comprises an antibody that specifically binds to the heavy chain constant region (e.g., the Fc region) and comprises a chemiluminescent label, and the composition further comprises a reagent that causes the chemiluminescent label to emit light.

In some embodiments, the composition may be a kit, further comprising instructions for using the kit.

The composition of the present application may comprise: i) the bispecific molecule of the present application, and ii) a T cell expressing a chimeric antigen receptor, wherein the chimeric antigen receptor comprises: a) an extracellular domain comprising a GS linker, b) a transmembrane domain, and c) an intracellular signalling domain. The chimeric antigen receptor may comprise the GS linker at any suitable position in the extracellular domain. In some embodiments, the extracellular domain of the chimeric antigen receptor may comprise a non-antigen-binding single-chain antibody that comprises a GS linker. In some embodiments, the extracellular domain of the chimeric antigen receptor may comprise a non-antigen-binding single-chain antibody, wherein the non-antigen-binding single-chain antibody may comprise a GS linker between the heavy chain variable region-like domain and light chain variable region-like domain thereof.

Alternatively, the composition of the present application may comprise: i) a T cell expressing the chimeric antigen receptor of the present application, and ii) an antibody or an antigen-binding portion thereof that is linked to a GS linker and targets a disease-associated antigen. The GS linker may be linked to the antibody or the antigen-binding portion thereof that targets the disease-associated antigen via a peptide, e.g., a peptide of 10 to 30 amino acids. The disease-associated antigen may be, for example, a tumour-associated antigen. The antibody or the antigen-binding portion thereof that targets a disease-associated antigen may be human-derived or humanized. The GS linker may be any GS linker, particularly a GS linker with a length of 6 or more amino acids, particularly a GS linker with a length of 6-20 amino acids, and particularly a GS linker with a length of 6-15 amino acids, including, but not limited to, (G₂S)₂, (G₂S)₄, (G₃S)₃, (G₄S)₂, and (G₄S)₃. In particular, the GS linker may be (G₄S)₃ or (G₄S)₂. The antibody or the antigen-binding portion thereof that targets a disease-associated antigen may not comprise a GS linker.

In another aspect, the present application provides a method for detecting a GS linker-containing molecule in a sample, the method comprising:
i) contacting the sample with the antibody or the antigen-binding portion thereof of the present application, and
ii) detecting the presence of the antibody or the antigen-binding portion thereof in the sample,
wherein the presence of the antibody or the antigen-binding portion thereof in the sample indicates the presence of a GS linker-containing molecule in the sample.

Alternatively, the present application provides a method for quantifying a GS linker-containing molecule in a sample, the method comprising:
i) contacting the sample with the antibody or the antigen-binding portion thereof of the present application, and
ii) detecting the presence of the antibody or the antigen-binding portion thereof in the sample,
wherein the GS linker-containing molecule in the sample is quantified on the basis of an amount of the antibody or the antigen-binding portion thereof in the sample.

Alternatively, the present application provides a method for purifying a GS linker-containing molecule from a sample, comprising:
i) contacting the sample with a solid support conjugated to the antibody or the antigen-binding portion thereof of the present application,
ii) separating from the sample the sample molecule bound to the solid support in step i) in the sample, and
iii) eluting from the solid support the sample molecule bound to the solid support,
wherein the solid support includes magnetic beads, resin, and agarose beads; and the sample molecule bound to the solid support is a GS linker-containing molecule.

The GS linker-containing molecule may be any GS linker-containing molecule, e.g., a recombinant protein or a nucleic acid-protein complex. In some embodiments, the GS linker-containing molecule may be a GS linker-containing antibody or a GS linker-containing chimeric antigen receptor. The GS linker-containing antibody may be a monospecific antibody, a bispecific antibody, or a multispecific antibody, and may comprise an scFv, a Fab, a nanobody, etc.

The GS linker may be any GS linker, particularly a GS linker with a length of 6 or more amino acids, particularly a GS linker with a length of 6-20 amino acids, and particularly a GS linker with a length of 6-15 amino acids, including, but not limited to, (G₂S)₂, (G₂S)₄, (G₃S)₃, (G₄S)₂, and (G₄S)₃. In particular, the GS linker may be (G₄S)₂ or (G₄S)₃.

In the method of the present application, one or more of the antibodies or the antigen-binding portions thereof of the present application may be used.

The method of the present application may further comprise, prior to step i), a step of selecting an antibody or an antigen-binding portion thereof on the basis of the GS linker.

When the GS linker is (G₂S)₂, an antibody or an antigen-binding portion thereof may be selected, wherein VH CDR1, VH CDR2, VH CDR3, VL CDR1, VL CDR2, and VL CDR3 comprise amino acid sequences set forth in: (1) NYDMY (SEQ ID NO: 2), VIYVNGNTHYASWAKG (SEQ ID NO: 6), GPF, QASQSVWKNKDLV (SEQ ID NO: 15), DASTLSS (SEQ ID NO: 21), and LGGFSCSRGDCGA (SEQ ID NO: 26), respectively; (2) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively; (3) NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively; or (4) NYDMY (SEQ ID NO: 2), VSYASGRTYSVRWAKG (SEQ ID NO: 10), GPL, QASQTVWKNNDLV (SEQ ID NO: 18), DASTLSS (SEQ ID NO: 21), and LGGFSCSSGDCGA (SEQ ID NO: 25), respectively.

When the GS linker is (G₂S)₄, an antibody or an antigen-binding portion thereof may be selected, wherein VH CDR1, VH CDR2, VH CDR3, VL CDR1, VL CDR2, and VL CDR3 comprise amino acid sequences set forth in: (1) NYDMY (SEQ ID NO: 2), VIYVNGNTHYASWAKG (SEQ ID NO: 6), GPF, QASQSVWKNKDLV (SEQ ID NO: 15), DASTLSS (SEQ ID NO: 21), and LGGFSCSRGDCGA (SEQ ID NO: 26), respectively; (2) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively; (3) NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively; or (4) NYDMY (SEQ ID NO: 2), VSYASGRTYSVRWAKG (SEQ ID NO: 10), GPL, QASQTVWKNNDLV (SEQ ID NO: 18), DASTLSS (SEQ ID NO: 21), and LGGFSCSSGDCGA (SEQ ID NO: 25), respectively.

When the GS linker is (G₃S)₃, an antibody or an antigen-binding portion thereof may be selected, wherein VH CDR1, VH CDR2, VH CDR3, VL CDR1, VL CDR2, and VL CDR3 comprise amino acid sequences set forth in: (1) NYAIM (SEQ ID NO: 1), VIYANGDPYCASWAKG (SEQ ID NO: 5), GGF, QSSQSIYNKNDLV (SEQ ID NO: 14), GISTLDS (SEQ ID NO: 20), and LGGFSCSSGDCGA (SEQ ID NO: 25), respectively; or (2) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively.

When the GS linker is (G₄S)₂, any one of the antibodies or the antigen-binding portions thereof described herein may be selected.

When the GS linker is (G₄S)₃, any one of the antibodies or the antigen-binding portions thereof described herein may be selected, particularly an antibody or an antigen-binding portion thereof, wherein VH CDR1, VH CDR2, VH CDR3, VL CDR1, VL CDR2, and VL CDR3 comprise amino acid sequences set forth in: (1) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively; (2) NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively; or (3) NYAIM (SEQ ID NO: 1), VIYANGDPYCASWAKG (SEQ ID NO: 5), GGF, QSSQSIYNKNDLV (SEQ ID NO: 14), GISTLDS (SEQ ID NO: 20), and LGGFSCSSGDCGA (SEQ ID NO: 25), respectively.

The sample may be any sample comprising a GS linker-containing molecule, e.g., a sample comprising a GS linker-containing antibody, protein sample or small molecule sample, or a cell sample comprising a GS linker-containing cell (e.g., a cell comprising a GS linker-containing CAR). In some embodiments, the sample may comprise cells treated with a GS linker-containing antibody. In some embodiments, the sample may comprise cells into which a vector has been introduced, wherein the vector comprises a sequence for expressing a GS linker-containing chimeric antigen receptor.

The antibody or the antigen-binding portion thereof of the present application may comprise a detectable substance. For example, the antibody or the antigen-binding portion thereof may be fused (e.g., by recombinant expression) with or conjugated to a detectable substance. The antibody or the antigen-binding portion thereof of the present application may comprise a detectable substance at any position that does not affect the antigen-binding property of the antibody or the antigen-binding portion thereof, e.g., in a heavy chain constant region, a light chain constant region, a framework region of the heavy chain variable region, or a framework region of the light chain variable region. In particular, the antibody or the antigen-binding portion thereof may comprise a detectable substance in a framework region of a heavy chain constant region or a light chain constant region. The detectable substance may be a fluorescent label or a chemiluminescent label. The fluorescent label may be fluorescein isothiocyanate. The chemiluminescent label may be peroxidase (e.g., horseradish peroxidase (HRP)) or alkaline phosphatase (AP).

In the method of the present application, the antibody or the antigen-binding portion thereof of the present application that comprises a fluorescent label may be used, and the presence or amount of the antibody or the antigen-binding portion thereof in the sample may be determined via a fluorescent signal.

In the method of the present application, the antibody or the antigen-binding portion thereof of the present application that comprises a chemiluminescent label may be used, the antibody or the antigen-binding portion thereof of the present application is contacted with a reagent that causes the chemiluminescent label to emit light, and the presence or amount of the antibody or the antigen-binding portion thereof in the sample is determined via a chemiluminescent signal. In some embodiments, in the method of the present application, an antibody or an antigen-binding portion thereof of the present application that comprises horseradish peroxidase may be used, and the antibody or the antigen-binding portion thereof is contacted with a peroxide (e.g., H₂O₂) and 3,3'-diaminobenzidine (DAB), or a peroxide (e.g., H₂O₂) and 3,3',5,5'-tetramethylbenzidine (TMB). In some embodiments, in the method of the present application, an antibody or an antigen-binding portion thereof of the present application that comprises alkaline phosphatase may be used, and the antibody or the antigen-binding portion thereof is contacted with 4-nitrophenyl phosphate disodium salt hexahydrate (pNPP).

The method of the present application can be used for detecting or quantifying a GS linker-containing protein, particularly the use in detecting the presence or amount of the GS linker-containing protein in a sample by, e.g., Western Blot; detecting the presence or amount of cells bound by the GS linker-containing protein in a sample by, e.g., flow cytometry; detecting the presence or amount of cells expressing the GS linker-containing protein in a sample by, e.g., flow cytometry; or detecting the presence or amount of cells expressing the GS linker-containing protein following viral transduction in a sample by, e.g., flow cytometry.

The present application also relates to a method for treating a disease in a subject in need thereof, the method comprising administering to the subject a composition of the present application, wherein the composition comprises: i) the bispecific molecule of the present application, and ii) a T cell expressing a chimeric antigen receptor, wherein the chimeric antigen receptor comprises: a) an extracellular domain comprising a GS linker, b) a transmembrane domain, and c) an intracellular signalling domain; or i) a T cell expressing the chimeric antigen receptor of the present application, and ii) an antibody or an antigen-binding portion thereof that is linked to a GS linker and targets a disease-associated antigen. The diseases may be associated with a disease-associated antigen targeted by a bispecific molecule in a composition, or by an antibody or an antigen-binding portion thereof that comprises a GS linker and targets the disease-associated antigen. The disease-associated antigen may be a tumour-associated antigen.

The present application also claims the use of the antibody or the antigen-binding portion thereof of the present application, or the composition of the present application in detecting or quantifying a GS linker-containing protein, particularly the use in detecting the presence or amount of the GS linker-containing protein in a sample by, e.g., Western Blot; detecting the presence or amount of cells bound by the GS linker-containing protein in a sample by, e.g., flow cytometry; detecting the presence or amount of cells expressing the GS linker-containing protein in a sample by, e.g., flow cytometry; or detecting the presence or amount of cells expressing the GS linker-containing protein following viral transduction in a sample by, e.g., flow cytometry.

The present application also claims the use of the bispecific molecule, the chimeric antigen receptor (CAR), the CAR-T cell, or the composition of the present application in treating a disease, or in the preparation of a drug for treating a disease.

It should be noted that, in the present application, particularly in the claims, terms such as "comprising" and "including" may have the meanings conferred under the Chinese Patent Law. Moreover, terms such as "consisting essentially of" have the meanings conferred under the Chinese Patent Law, for example, permitting the presence of elements not expressly recited, but excluding elements that exist in the prior art or that affect the basic or novel characteristics of the present disclosure.

Other features and advantages of the present disclosure will become more apparent with reference to the following detailed description and examples, which shall not be construed as limiting. The content of all of the documents, Genbank records, patents, and published patent applications cited in the present application is expressly incorporated herein by reference.

### BRIEF DESCRIPTION OF THE DRAWINGS

The following detailed description is provided by way of example, but is not intended to limit the present disclosure to the specific embodiments described, and can be better understood with reference to the accompanying drawings.
FIG. 1 shows the binding affinity of the antibodies R177.B8 (A), R177.B11 (B), R177.D13 (C), R177.E4 (D), R177.G3 (E), R177.G5 (F), R177.G12 (G), and R177.I8 (H) of the present application for various GS linkers in ELISA assays;
FIG. 2 shows the binding of the antibodies R177.B8 (A), R177.B11 (B), R177.G3 (C), R177.G5 (D), R177.D13 (E), R177.E4 (F), R177.G12 (G), and R177.I8 (H) of the present application to various GS linkers in Western Blot assays;
FIG. 3 shows the percentage of cells bound with GS linker-containing antibodies detected by the antibodies of the present application relative to total cells;
FIG. 4 shows lentivirus-transduced cells detected by the antibodies of the present application;
FIG. 5 shows cells expressing a GS linker-containing chimeric antigen receptor detected by the antibodies of the present application; and
FIG. 6 shows the SDS-PAGE electrophoresis result of scFv containing a GS linker purified by the antibodies of the present application.

### DETAILED DESCRIPTION

Unless otherwise indicated, the terms used herein have ordinary meanings as set forth in dictionaries, textbooks and technical references, or as commonly understood by those skilled in the art. The following descriptions of certain terms are provided only for the convenience of understanding the present application and are not intended to impose special limitations on such terms unless otherwise indicated.

As used herein and in the appended claims, the singular forms "a", "an", and "the" include plural referents unless otherwise clearly specified in the context.

The term "or" refers to a single element from the list of selectable elements unless otherwise clearly indicated in the context.

The term "comprise" or "include" refers to the inclusion of the stated elements, integers, or steps, but not to the exclusion of any other elements, integers, or steps. The term "comprise" or "include" used herein, unless indicated, also encompasses combinations of the stated elements, integers, or steps.

"GS linker" refers to a peptide composed of glycine and serine in the form of (GnS)m, where n and m may be integers of at least 1. In particular, the GS linker in the present application refers to a GS linker with a length of 6 or more amino acids, particularly a GS linker with a length of 6-20 amino acids, and particularly a GS linker with a length of 6-15 amino acids, including, but not limited to, (G₂S)₂, (G₂S)₄, (G₃S)₃, (G₄S)₂, and (G₄S)₃.

The "detectable substance" in the present application refers to a substance or molecule comprising a group detectable by the naked eye or an instrument. The group detectable by the naked eye or an instrument may be, for example, a fluorescent label, a luminescent label, an immunodetectable label, a radioactive label, a chemical label, a nucleic acid label, or a polypeptide label. Therefore, the detectable substance may be a fluorescent marker containing a fluorescent group, or a chemiluminescent marker containing a group capable of direct colour development or enzymatic colour development. The antibody or the antigen-binding portion thereof of the present application may be localized or quantified via a detectable substance contained (e.g., conjugated) on the antibody or the antigen-binding portion thereof.

The "conjugation" in the present application may refer to a state in which two organic chemical groups are linked via a certain chemical reaction, or a state in which two substances are attached to each other via any reaction or any chemical bond. For example, the detectable substance may be linked to the antibody or the antigen-binding portion thereof by reacting with a side chain group on a certain amino acid of the antibody or the antigen-binding portion thereof, or the detectable substance may be attached to the antibody or the antigen-binding portion thereof via, for example, van der Waals bonds.

The "non-antigen-binding single-chain antibody" in the present application refers to an scFv-like peptide that has the structure of a single-chain antibody, i.e., heavy chain variable region-linker-light chain variable region, but does not specifically bind to any disease-associated antigen. The "non-antigen-binding single-chain antibody" may comprise a heavy chain variable region-like peptide (i.e., a peptide with an amino acid length similar to that of an antibody heavy chain variable region), a GS linker, and a light chain variable region-like peptide (i.e., a peptide with an amino acid length similar to that of an antibody light chain variable region).

The term "antibody" herein is intended to include full-length antibodies of IgG, IgA, IgD, IgE, and IgM and any antigen-binding fragment (i.e., antigen-binding portion). A full-length antibody is a glycoprotein comprising at least two heavy (H) chains and two light (L) chains, the heavy chain and the light chain being linked by disulfide bonds. Each heavy chain consists of a heavy chain variable region (abbreviated as V_{H} or VH) and a heavy chain constant region. The heavy chain constant region consists of three domains: C_{H1}, C_{H2}, and C_{H3}. Each light chain consists of a light chain variable region (abbreviated as V_{L} or VL) and a light chain constant region. The light chain constant region consists of one domain, C_{L}. The V_{H} and V_{L} regions may also be divided into hypervariable regions, known as complementarity-determining regions (CDRs), which are separated by more highly conserved framework regions (FRs). Each V_{H} or V_{L} consists of three CDRs and four FRs arranged from the amino terminus to the carboxyl terminus in the order of FR1, CDR1, FR2, CDR2, FR3, CDR3, and FR4. The variable regions of the heavy chains and light chains comprise binding domains that interact with antigens. The constant regions of the antibody can mediate the binding of immunoglobulins to host tissues or factors, including binding to various cells of the immune system (e.g., effector cells) and the first component (C1q) of the classical complement system. A "functional fragment" of an antibody constant region refers to a fragment of the constant region that retains certain desired functions, e.g., an Fc fragment in a heavy chain constant region.

The term "antigen-binding portion" of an antibody (or abbreviated as an antibody portion) herein refers to one or more fragments of an antibody that retain the ability to specifically bind to an antigen (e.g., GS linker). It has been demonstrated that the antigen-binding functionality of an antibody can be implemented by fragments of a full-length antibody. Examples of binding fragments comprised in the "antigen-binding portion" of an antibody include: (i) a Fab fragment, a monovalent fragment consisting of V_{L}, V_{H}, C_{L}, and C_{H1}; (ii) an F(ab')₂ fragment, a bivalent fragment comprising two Fab fragments linked by a disulfide bridge in the hinge region; (iii) an Fd fragment consisting of V_{H} and C_{H1}; (iv) an scFv fragment consisting of the V_{L} and V_{H} of a single arm of an antibody; (v) a dAb fragment (Ward et al., (1989) Nature 341: 544-546) consisting of V_{H}H; (vi) an isolated complementarity-determining region (CDR); and (vii) a dAb-V_{L}, a fragment comprising a single variable domain and a heavy chain constant domain. Furthermore, although the two domains of the scFv fragment, V_{L} and V_{H}, are encoded by different genes, they can be linked by recombinant methods via a synthetic linker that brings the two into a single protein chain, in which the V_{L} and V_{H} regions are paired to form a monovalent molecule. These single-chain antibodies are also intended to be included within the meaning of the term. These antibody fragments can be obtained by conventional techniques known to those skilled in the art, and the fragments can be functionally screened in the same manner as intact antibodies.

"Single-chain antibody" or "scFv" refers to an antibody form in which the heavy chain variable region of an antibody is linked to the light chain variable region directly or via a linker.

"Nanobody" or "V_{H}H" refers to a single antigen-binding polypeptide comprising a single monomeric variable region, which is capable of binding to an antigen without pairing with another corresponding CDR-containing polypeptide, wherein the variable region comprises three complementarity determining regions (CDRs).

The term "isolated antibody" as used herein refers to an antibody that is substantially free of other antibodies having different antigen specificities. For example, an isolated antibody that specifically binds to a GS linker protein is substantially free of antibodies that specifically bind to antigens other than the GS linker. The isolated antibody is substantially free of other cellular materials and/or chemical substances.

The term "monoclonal antibody" or "mAb" or "monoclonal antibody composition" refers to an antibody molecule product consisting of a single molecule. The monoclonal antibody composition exhibits single binding specificity and affinity for a specific epitope.

The term "rabbit antibody" refers to an antibody in which the variable region framework and CDR regions are derived from rabbit germline immunoglobulin sequences. Furthermore, if the antibody comprises a constant region, the constant region is also derived from rabbit germline immunoglobulin sequences. The rabbit antibody of the present application may comprise amino acid residues that are not encoded by rabbit germline immunoglobulin sequences, such as mutations introduced by random or site-directed mutagenesis *in vitro*, or by somatic mutation *in vivo*. However, the term "rabbit antibody" does not include antibodies in which CDR sequences derived from other mammalian species have been inserted into rabbit framework sequences.

The term "chimeric antibody" refers to an antibody that combines genetic material from one species with genetic material from another species. In particular, a chimeric antibody in the present application refers to an antibody obtained by combining non-human genetic material with human genetic material.

The term "humanized antibody" refers to an antibody derived from a non-human (e.g., rabbit) species, of which the protein sequence has been modified to increase its similarity to antibodies naturally produced in humans.

The terms "antibody recognizing an antigen" and "antibody specific for an antigen" are used interchangeably herein with the term "antibody specifically binding to an antigen".

Herein, the term "specifically recognizes" or "specifically binds to" a target, e.g., a GS linker, means that an antibody or an antigen-binding fragment is capable of distinguishing the GS linker from one or more reference molecules, e.g., other amino acid peptides, and exhibits binding affinity or binding activity for the GS linker that is, for example, 1-fold, 5-fold, 10-fold or higher than that for other reference molecules. Methods for determining specificity include, but are not limited to, SPR, Western blotting, ELISA, RIA, ECL, IRMA assays, and peptide scanning.

The term "sequence identity" herein refers to the percentage of nucleotides/amino acids in a sequence that are identical to the nucleotides/amino acid residues in a reference sequence after a sequence alignment, and if desired, gaps are introduced in the sequence alignment to achieve the maximum percent sequence identity between the two sequences. Those skilled in the art can perform pairwise sequence alignment or multiple sequence alignment using various methods, e.g., computer software, to determine the percent sequence identity between two or more nucleic acid or amino acid sequences. Examples of such computer software include Clustal Omega, T-Coffee, Kalign, MAFFT, etc.

"Chimeric antigen receptor" or "CAR" refers to a recombinant protein comprising both antigen-recognition function and immune cell-activating function, which is generally recombinantly expressed in immune cells, such that the immune cells exert cytotoxicity against cells harbouring the target antigen, e.g., tumour cells, upon recognizing and binding to the target antigen. A CAR generally comprises (a) an extracellular antigen-binding domain, (b) a transmembrane domain, and (c) an intracellular signalling domain, wherein the extracellular antigen-binding domain comprises an scFv or nanobody that specifically binds to the target antigen. In some embodiments of the present application, the extracellular antigen-binding domain may comprise a GS linker, which may be located, for example, in an scFv or between two or more nanobodies.

The term "EC₅₀", also known as half-maximal effective concentration, refers to the concentration of a drug that induces 50% of the maximal effect.

The term "IC₅₀" refers to half-maximal inhibitory concentration, i.e., the concentration of a drug or inhibitor required to inhibit a specified biological process by half.

The term "subject" includes any human or non-human animal. The term "non-human animal" includes all vertebrates, such as mammals and non-mammals, such as non-human primates, sheep, dogs, cats, cows, horses, chickens, amphibians, and reptiles; although mammals such as non-human primates, sheep, dogs, cats, cows, and horses are preferred.

The term "therapeutically effective amount" refers to an amount of the molecule of the present application sufficient to prevent or alleviate symptoms associated with a disease or disorder (e.g., cancer). The therapeutically effective amount is dependent on the disease being treated, and the actual effective amount can be readily determined by those skilled in the art.

The antibody or the antigen-binding portion thereof of the present application that specifically binds to a GS linker is capable of binding to various GS linkers with high affinity, including, but not limited to, (G₂S)₂, (G₂S)₄, (G₃S)₃, (G₄S)₂, and (G₄S)₃. In particular, the antibody or the antigen-binding portion thereof of the present application is capable of specifically binding to (G₄S)₂ or (G₄S)₃.

Compared with conventional methods using protein L, target antigens, anti-idiotypic antibodies, etc., the antibody or the antigen-binding portion thereof of the present application: i) has a certain degree of universality, i.e., is applicable to most GS linker-containing proteins regardless of the target of the protein, the subtype of the Fc region contained in the protein, the subtype of the antibody light chain, the antibody form (such as single-domain antibody or scFv), etc., and is applicable to most GS linkers other than G₄S; ii) has high binding specificity for GS linker-containing proteins, with higher detection accuracy than the protein L assay, and stronger discrimination than anti-Fab antibodies between samples with GS linker-containing proteins and samples without GS linker-containing proteins, e.g., positive and negative CAR-T cells with or without a GS linker-containing scFv; and iii) has high binding sensitivity to GS linker-containing proteins, enabling the detection of about 2 pmol of a GS linker-containing protein. In particular, the antibody of the present application is a monoclonal antibody. Furthermore, the antibody or the antigen-binding portion thereof may be, e.g., rabbit-derived, chimeric, or humanized.

The sequences or sequence numbers of the heavy chain variable regions and the light chain variable regions of the antibodies or the antigen-binding portions thereof of the present application are listed in Table 1. The CDRs of the heavy chain variable region and the CDRs of the light chain variable region are determined according to the Kabat numbering system, and the CDR sequences or sequence numbers thus determined are listed in Table 1. The CDRs of the heavy chain variable region and the CDRs of the light chain variable region of the antibodies or the antigen-binding portions thereof of the present application may also be determined according to the IMGT, Chothia, AbM, or Contact numbering system based on the full-length variable region sequences.

V_{H} and/or V_{L} sequences (or CDR sequences) of other antibodies that bind to the GS linker may be "mixed and matched" with the V_{H} and/or V_{L} sequences (or CDR sequences) of the antibodies of the present application. Preferably, when V_{H} and V_{L} (or CDRs therein) are mixed and matched, the V_{H} sequence in a specific V_{H}/V_{L} pair can be replaced by a structurally similar V_{H} sequence. Similarly, preferably, the V_{L} sequence in a specific V_{H}/V_{L} pair is replaced by a structurally similar V_{L} sequence.

Therefore, in one embodiment, the antibody or the antigen-binding portion thereof of the present application comprises:
(a) a heavy chain variable region comprising an amino acid sequence listed in Table 1; and
(b) a light chain variable region comprising an amino acid sequence listed in Table 1, or a V_{L} of another GS linker antibody, wherein the antibody specifically binds to a GS linker.

In another embodiment, the antibody or the antigen-binding portion thereof of the present application comprises:
(a) CDR1, CDR2, and CDR3 of the heavy chain variable region listed in Table 1; and
(b) CDR1, CDR2, and CDR3 of the light chain variable region listed in Table 1, or CDRs of another GS linker antibody, wherein the antibody specifically binds to a GS linker.

In another embodiment, the antibody or the antigen-binding portion thereof of the present application comprises heavy chain variable region CDR2 of a GS linker antibody, and CDRs of other GS linker-binding antibodies, e.g., heavy chain variable region CDR1, and/or CDR3, and/or light chain variable region CDR1, CDR2, and/or CDR3 of another GS linker antibody.

Furthermore, it is well known in the art that the CDR3 domain, independently of CDR1 and/or CDR2, can alone determine the binding specificity of antibodies for the same antigen, and it can be predicted that multiple antibodies having the same binding specificity can be generated based on the CDR3 sequence. See, e.g., Klimka et al., British J. of Cancer 83(2): 252-260 (2000); Beiboer et al., J. Mol. Biol. 296: 833-849 (2000); Rader et al., Proc. Natl. Acad. Sci. U.S.A. 95: 8910-8915 (1998); Barbas et al., J. Am. Chem. Soc. 116: 2161-2162 (1994); Barbas et al., Proc. Natl. Acad. Sci. U.S.A. 92: 2529-2533 (1995); Ditzel et al., J. Immunol. 157: 739-749 (1996).

In another embodiment, the antibody or the antigen-binding portion thereof of the present application comprises heavy and/or light chain variable region sequences, or CDR1, CDR2, and CDR3 sequences, with one or more conservative modifications relative to those of the GS linker antibody or the antigen-binding portion thereof of the present application. It is known in the art that some conservative sequence modifications do not eliminate the antigen-binding affinity. See, e.g., Brummell et al., (1993) Biochem 32: 1180-1188.

Therefore, in one embodiment, the antibody or the antigen-binding portion thereof comprises a heavy chain variable region and/or a light chain variable region, wherein the heavy chain variable region and the light chain variable region each comprise CDR1, CDR2, and CDR3, wherein:
(a) CDR1 of the heavy chain variable region comprises a sequence listed in Table 1, and/or a conservative modification thereof; and/or
(b) CDR2 of the heavy chain variable region comprises a sequence listed in Table 1, and/or a conservative modification thereof; and/or
(c) CDR3 of the heavy chain variable region comprises a sequence listed in Table 1, and/or a conservative modification thereof; and/or
(d) CDR1, and/or CDR2, and/or CDR3 of the light chain variable region comprise a sequence listed in Table 1, and/or a conservative modification thereof; and
(e) the antibody or the antigen-binding portion thereof specifically binds to a GS linker.

The term "conservative sequence modification" as used herein refers to an amino acid modification that does not significantly affect or alter the binding properties of the antibody. Such conservative modifications include amino acid substitutions, additions and deletions. Modifications can be introduced into the antibody of the present application or the antigen-binding portion thereof by standard techniques known in the art, e.g., point mutations and PCR-mediated mutations. Conservative amino acid substitutions are those in which an amino acid residue is substituted with an amino acid residue having a similar side chain. Groups of amino acid residues having similar side chains are known in the art. Such groups of amino acid residues include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in the CDR regions of the antibody or the antigen-binding portion thereof of the present application can be substituted with other amino acid residues from the same side chain group, and the resulting antibody can be tested for retained function (i.e., the functions described above) using the functional assays described herein.

The antibody or the antigen-binding portion thereof of the present application can be prepared as a genetically modified antibody using, as a starting material, an antibody having one or more V_{H}/V_{L} sequences of the GS linker antibody or the antigen-binding portion thereof of the present application. The antibody can be genetically modified by modifying one or more residues in one or both variable regions (i.e., V_{H} and/or V_{L}) (e.g., in one or more CDR regions and/or one or more framework regions) to improve binding affinity.

Variable region modifications may involve mutating amino acid residues in the V_{H} and/or V_{L} CDR1, CDR2, and/or CDR3 regions, thereby improving one or more binding properties (e.g., affinity) of the antibody of interest. Point mutations or PCR-mediated mutations can be performed to introduce mutations, and the effects thereof on antibody binding or other functional properties can be assessed in *in vitro* or *in vivo* assays known in the art. Preferably, conservative modifications known in the art are introduced. The mutations may be amino acid substitutions, additions, or deletions, but substitutions are preferred. Furthermore, generally no more than one, two, three, four, or five residues in the CDR regions are altered.

Furthermore, in another embodiment, the present application provides an isolated GS linker monoclonal antibody or an antigen-binding portion thereof, comprising a heavy chain variable region and a light chain variable region, which comprise: (a) a V_{H} CDR1 region comprising a sequence of the present application, or an amino acid sequence having one, two, three, four, or five amino acid substitutions, deletions, or additions; (b) a V_{H} CDR2 region comprising a sequence of the present application, or an amino acid sequence having one, two, three, four, or five amino acid substitutions, deletions, or additions; (c) a V_{H} CDR3 region comprising a sequence of the present application, or an amino acid sequence having one, two, three, four, or five amino acid substitutions, deletions, or additions; (d) a V_{L} CDR1 region comprising a sequence of the present application, or an amino acid sequence having one, two, three, four, or five amino acid substitutions, deletions, or additions; (e) a V_{L} CDR2 region comprising a sequence of the present application, or an amino acid sequence having one, two, three, four, or five amino acid substitutions, deletions, or additions; and (f) a V_{L} CDR3 region comprising a sequence of the present application, or an amino acid sequence having one, two, three, four, or five amino acid substitutions, deletions, or additions.

The hinge region of C_{H1} of the antibody is modified to alter, e.g., to increase or decrease, the number of cysteine residues in the hinge region. This method is further described in U.S. Pat. No. 5,677,425. Cysteine residues in the C_{H1} hinge region are altered, for example, to facilitate the assembly of the heavy chain and light chain or to increase/decrease stability of the antibody.

The glycosylation of the antibody can be modified. For example, deglycosylated antibodies (i.e., antibodies lacking glycosylation) can be prepared. Glycosylation can be altered, for example, to increase the affinity of the antibody for an antigen. Such glycosylation modifications can be achieved, for example, by altering one or more glycosylation sites in the sequence of the antibody. For example, one or more amino acid substitutions can be made to eliminate one or more framework glycosylation sites in the variable regions, thereby eliminating glycosylation at these sites. Such deglycosylation may increase the affinity of the antibody for the antigen. See, e.g., U.S. Pat. Nos. 5,714,350 and 6,350,861. Glycosylation is known to occur in motifs containing N-X-S/T sequences. In some cases, it is preferred that the GS linker antibody or the antigen-binding portion thereof does not contain variable region glycosylation. This can be achieved by selecting an antibody that does not contain glycosylation motifs in the variable regions, or by mutating residues in the glycosylation region.

In preferred embodiments, the antibody or the antigen-binding portion does not contain an asparagine isomerization site. Deamidation of asparagine may occur in the N-G or D-G sequence, creating isoaspartate residues that introduce a kink into the polypeptide chain and reduce its stability (the isoaspartate effect).

The monoclonal antibodies of the present application can be prepared using phage display technology. Phage display technology involves inserting a gene encoding an exogenous polypeptide or protein (e.g., an antibody in the form of an scFv) into an appropriate position of the phage coat protein structural gene by genetic engineering techniques, such that the exogenous polypeptide or protein is correctly expressed in the reading frame. As a result, the exogenous polypeptide or protein (e.g., scFv) forms a fusion protein on the phage capsid protein and is displayed on the phage surface upon reassembly of progeny phages. Subsequently, by using a target molecule (e.g., GS linker) and adopting an appropriate panning method, phages that do not specifically bind to the target molecule are washed away. The bound phages are then eluted using an acid, base, or competing molecule, and the neutralized phages are used to infect Escherichia coli for amplification. After 3 to 5 rounds of enrichment, the proportion of phages capable of specifically recognizing the target molecule is gradually increased, and finally polypeptides or proteins that recognize the target molecule are obtained.

Other methods for preparing the monoclonal antibodies include somatic cell hybridization (hybridoma), viral or oncogenic transformation of B lymphocytes, etc. Methods for preparing chimeric antibodies are also well known in the art. The antibodies or the antigen-binding portions thereof of the present application can also be produced in host cell transfectomas using, for example, recombinant DNA techniques in conjunction with gene transfection methods (see, e.g., Morrison, S. (1985) Science 229: 1202). In one embodiment, DNA encoding partial or full-length light and heavy chains obtained by standard molecular biology techniques is inserted into one or more expression vectors such that the genes are operably linked to transcriptional and translational regulatory sequences. In this case, the term "operably linked" refers to the linkage of the antibody genes into the vector such that the transcriptional and translational control sequences within the vector perform their intended function of regulating the transcription and translation of the antibody genes.

The term "regulatory sequence" includes promoters, enhancers, and other expression control elements (e.g., polyadenylation signals) that control the transcription or translation of the antibody genes. Preferred regulatory sequences for expression in mammalian host cells include viral elements that direct high-level protein expression in mammalian cells, e.g., promoters and/or enhancers derived from cytomegalovirus (CMV), simian virus 40 (SV40), adenovirus such as the adenovirus major late promoter (AdMLP), and polyomavirus. Alternatively, non-viral regulatory sequences can be used, such as the ubiquitin promoter or the β-globin promoter. In addition, the regulatory elements consist of sequences from different sources. For example, the SRα promoter system comprises sequences from the SV40 early promoter and the long terminal repeat of human T-cell leukemia virus type I. The expression vector and expression control sequences are selected to be compatible with the expression host cell used

The antibody light chain gene and the antibody heavy chain gene can be inserted into the same expression vector or different expression vectors. In preferred embodiments, variable regions are inserted into an expression vector encoding the heavy chain constant region and the light chain constant region of the desired subtype to construct a full-length antibody gene, such that the V_{H} is operably linked to the C_{H} in the vector and the V_{L} is operably linked to the C_{L} in the vector. Alternatively, the recombinant expression vector can encode a signal peptide that facilitates the secretion of antibody chains from the host cell. The antibody chain gene can be cloned into the vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector of the present application may carry other sequences, e.g., a sequence that regulates the replication of the vector in the host cell (e.g., an origin of replication) and a selectable marker gene. The selectable marker gene can be used to select a host cell into which the vector has been introduced. For example, the selectable marker gene generally confers drug resistance to the host cell into which the vector has been introduced, e.g., resistance to G418, hygromycin, or methotrexate. Preferred selectable marker genes include the dihydrofolate reductase (DHFR) gene (for methotrexate selection/amplification in dhfr host cells) and the neo gene (for G418 selection).

For the expression of light and heavy chains, expression vectors encoding the heavy and light chains are transfected into host cells by standard techniques. The term "transfection" in its various forms encompasses a variety of techniques commonly used to introduce exogenous DNA into prokaryotic or eukaryotic host cells, e.g., electroporation, calcium phosphate precipitation, DEAE-dextran transfection, etc. Although the expression of the antibody or the antigen-binding portion thereof of the present application in prokaryotic or eukaryotic host cells is theoretically feasible, the antibody is preferably expressed in eukaryotic cells, and most preferably in mammalian host cells, because eukaryotic cells, particularly mammalian cells, are more likely than prokaryotic cells to assemble and secrete appropriately folded and immunologically active antibodies.

Preferred mammalian host cells for expressing the recombinant antibodies of the present application include Slc35C1 knockout cell lines, FUT8 knockout cell lines, the variant CHO cell line Lec13, the rat myeloma cell line YB2/0, cell lines comprising small interfering RNAs specifically directed against the FUT8 gene, cell lines co-expressing β-1,4-N-acetylglucosaminyltransferase III and Golgi α-mannosidase II, Chinese hamster ovary (CHO) cells (including dhfr⁻ CHO cells used with the DHFR selectable marker), NSO myeloma cells, COS cells, and SP2 cells. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the culture medium in which the host cell is grown. The antibody or the antigen-binding portion thereof can be recovered from the culture medium using protein purification methods.

In certain embodiments, CDR grafting can be used to genetically modify variable regions of an antibody. The antibody mainly interacts with a target antigen through amino acid residues located in the three heavy chain complementarity determining regions (CDRs) and the six light chain CDRs. For this reason, amino acid residues in CDRs are more diverse between individual antibodies than sequences outside the CDRs. Since the CDR sequences are responsible for the major antibody-antigen interactions, recombinant antibodies simulating the properties of a particular natural antibody can be expressed by constructing expression vectors containing CDR sequences of the particular natural antibody grafted into the framework sequences of different antibodies with different properties (Riechmann et al., (1998) Nature 332:323-327; Jones et al., (1986) Nature 321:522-525; Queen et al., (1989) Proc. Natl. Acad; U.S.A. 86:10029-10033; U.S. Pat. Nos. 5,225,539; 5,530,101; 5,585,089; 5,693,762; and 6,180,370).

Therefore, another embodiment of the present application relates to an isolated monoclonal antibody or an antigen-binding fragment thereof, and/or a bispecific antibody, which comprises a heavy chain variable region and/or a light chain variable region, wherein the heavy chain variable region comprises CDR1, CDR2, and CDR3 having the sequences of the present application, and the light chain variable region comprises CDR1, CDR2, and CDR3 having the sequences of the present application. Although these antibodies comprise the V_{H} and V_{L} CDR sequences of the antibodies of the present application, they may contain different framework sequences.

Such framework sequences can be found in public DNA databases or public references including germline antibody gene sequences. For example, germline DNA sequences for human heavy and light chain variable region genes are available in the Vbase human germline sequence database (www.mrc-cpe.cam.ac.uk/vbase), as well as in Kabat et al., (1991), supra.; Tomlinson et al., (1992) J. Mol. Biol. 227: 776-798; and Cox et al., (1994) Eur. J. Immunol. 24: 827-836. As another embodiment, germline DNA sequences for human heavy and light chain variable region genes are available in the Genbank database.

The antibody protein sequences are compared against protein sequence databases using one of the sequence similarity search methods commonly known in the art as gap BLAST (Altschul et al., 1997).

Preferred framework sequences for the antibody of the present application are those that are structurally similar to the framework sequences used in the antibody of the present application. The V_{H} CDR1, CDR2, and CDR3 sequences may be grafted into framework regions having the same sequence as the germline immunoglobulin gene from which the framework sequence is derived, or the CDR sequences may be grafted into framework regions containing one or more mutations as compared to the germline sequence. For example, in some cases, it is beneficial to mutate residues in the framework regions to maintain or enhance the antigen-binding property of the antibody (see, e.g., U.S. Pat. Nos. 5,530,101; 5,585,089; 5,693,762; and 6,180,370).

In another aspect, the present application provides a nucleic acid molecule encoding the heavy chain and/or light chain variable regions or CDRs of the antibody or the antigen-binding portion thereof of the present application. The nucleic acid may be present in an intact cell, in a cell lysate, or in a partially purified or substantially pure form. The nucleic acid is "isolated" or "substantially pure" when purified from other cellular components or other contaminants, such as other cellular nucleic acids or proteins, by standard techniques. The nucleic acid molecules of the present application may be, for example, DNA or RNA, and may or may not comprise intron sequences. In preferred embodiments, the nucleic acid is a cDNA molecule.

The nucleic acid of the present application can be obtained using standard molecular biology techniques. For antibodies expressed by hybridomas (e.g., hybridomas prepared from transgenic mice carrying human immunoglobulin genes), cDNAs encoding the light and heavy chains of the antibodies prepared from the hybridomas can be obtained by standard PCR amplification or cDNA cloning techniques. For antibodies obtained from an immunoglobulin gene library (e.g., using phage display technology), nucleic acids encoding such antibodies can be collected from the gene library.

Preferably, the nucleic acid molecules of the present application include those encoding the V_{H} and V_{L} sequences or CDRs of the GS linker-containing monoclonal antibody. Once the DNA fragments encoding the V_{H} and V_{L} are obtained, operations such as conversion of the variable region genes to full-length antibody chain genes, Fab fragment genes, or scFv genes can be further performed on these DNA fragments by standard recombinant DNA techniques. In these operations, the DNA fragment encoding the V_{H} or V_{L} is operably linked to another DNA fragment encoding another protein, such as an antibody constant region or a flexible linker. The term "operably linked" means that two DNA fragments are linked together such that the amino acid sequences encoded by the two DNA fragments are both in the reading frame.

An isolated DNA encoding the V_{H} region can be converted into a full-length heavy chain gene by operably linking the V_{H}-encoding DNA to another DNA molecule encoding heavy chain constant region (C_{H1}, C_{H2}, and C_{H3}). The sequences of the human heavy chain constant region genes are known in the art, and DNA fragments comprising these regions can be obtained by standard PCR amplification. The heavy chain constant region may be an IgG1, IgG2, IgG3, IgG4, IgA, IgE, IgM, or IgD constant region, but is preferably an IgG1 constant region. For Fab fragment heavy chain genes, DNA encoding the V_{H} region can be operably linked to another DNA molecule encoding only the heavy chain C_{H1} constant region.

An isolated DNA encoding the V_{L} region can be converted into a full-length light chain gene by operably linking the V_{L}-encoding DNA to another DNA molecule encoding the light chain constant region C_{L}. The sequences of the human light chain constant region genes are known in the art, and DNA fragments comprising these regions can be obtained by standard PCR amplification. In preferred embodiments, the light chain constant region may include κ and λ constant regions.

To create an scFv gene, DNA fragments encoding V_{H} and V_{L} can be operably linked to another fragment encoding a flexible linker, such that the V_{H} and V_{L} sequences can be expressed as a contiguous single-chain protein, in which the V_{H} and V_{L} regions are linked via the flexible linker.

In another aspect, the present application relates to a bispecific molecule comprising the antibody or the antigen-binding portion thereof of the present application linked to at least one other functional molecule, such as another peptide or protein (e.g., another antibody or a receptor ligand), to generate a bispecific molecule that binds to at least two different binding sites or target molecules. The term "bispecific molecule" includes molecules having three or more specificities. In some embodiments, the bispecific molecule of the present application may comprise: i) the antibody or the antigen-binding portion thereof of the present application that specifically binds to a GS linker, and ii) an antibody or an antigen-binding portion thereof that targets a disease-associated antigen. i) and ii) may be linked together, for example, fused together by recombinant expression, or conjugated together via, e.g., a disulfide bond. The disease-associated antigen may be, for example, a tumour-associated antigen.

Bispecific molecules may be present in a variety of forms and sizes. At one end of the size spectrum, bispecific molecules remain in the conventional antibody form, except that they have two binding arms with different types of specificity instead of having two binding arms with the same specificity. At the other extreme are bispecific molecules consisting of two single-chain antibody fragments (scFvs) linked via a peptide chain, referred to as Bs(scFv)₂ constructs. Bispecific molecules of intermediate size comprise two different F(ab) fragments linked via a peptide linker. These and other forms of bispecific molecules can be prepared by genetic engineering, somatic hybridization, or chemical methods.

The present application further provides a chimeric antigen receptor, which may comprise: a) an extracellular domain, which may comprise the antibody or the antigen-binding portion thereof of the present application that specifically binds to a GS linker, particularly the antibody of the present application in the form of an scFv; b) a transmembrane domain; and c) an intracellular signalling domain.

The present application further provides an immune cell, such as a T cell or NK cell, comprising the chimeric antigen receptor of the present application.

To facilitate the detection of the antibody or the antigen-binding portion thereof of the present application, the antibody or the antigen-binding portion thereof may comprise a detectable substance. For example, the antibody or the antigen-binding portion thereof may be fused (e.g., by recombinant expression) with or conjugated to a detectable substance. The antibody or the antigen-binding portion thereof of the present application may comprise a detectable substance in a heavy chain constant region, a light chain constant region, a framework (FR) region of the heavy chain variable region, or a framework region of the light chain variable region. In particular, the antibody or the antigen-binding portion thereof may comprise a detectable substance in a framework region of a heavy chain constant region or a light chain constant region.

The detectable substance may be any substance readily detectable by the naked eye or by an instrument, including, but not limited to, a fluorescent label, a luminescent label, an immunodetectable label, a radioactive label, a chemical label, a nucleic acid label, and a polypeptide label. Commonly used detectable substances include fluorescent labels or chemiluminescent labels. The fluorescent label may be, for example, fluorescein isothiocyanate (FITC), which can be qualitatively and quantitatively observed by, e.g., fluorescence microscopy, and can be conveniently used in, e.g., flow cytometry. The chemiluminescent label may be peroxidase (e.g., horseradish peroxidase (HRP)), alkaline phosphatase (AP), etc., which can rapidly develop colour upon addition of the substrate corresponding to each enzyme, thereby enabling qualitative and quantitative observation. Substrates for horseradish peroxidase include 3,3'-diaminobenzidine (DAB) and 3,3',5,5'-tetramethylbenzidine (TMB). Substrates for alkaline phosphatase include 4-nitrophenyl phosphate disodium salt hexahydrate (pNPP).

Methods for incorporating a detectable substance into the antibody or the antigen-binding portion thereof of the present application, e.g., conjugation or recombinant expression of fusion proteins, are known to those skilled in the art.

The antibody or the antigen-binding portion thereof of the present application can also be qualitatively and quantitatively observed by addition of a secondary antibody that binds to the heavy chain constant region or light chain constant region thereof, particularly the heavy chain constant region such as the Fc region.

In another aspect, the present application provides a composition comprising the antibody or the antigen-binding portion thereof, the bispecific molecule, the chimeric antigen receptor (CAR), the cell carrying the CAR, the nucleic acid molecule, the expression vector, or the host cell of the present application.

The antibody or the antigen-binding portion thereof of the present application may comprise a detectable substance, e.g., a fluorescent label or a chemiluminescent label. In some embodiments, the antibody or the antigen-binding portion thereof of the present application may comprise a chemiluminescent label, and the composition may further comprise a reagent that causes the chemiluminescent label to emit light. In some embodiments, the chemiluminescent label may be horseradish peroxidase, and the reagent that causes the chemiluminescent label to emit light may be a peroxide (e.g., H₂O₂) and 3,3'-diaminobenzidine (DAB), or a peroxide (e.g., H₂O₂) and 3,3',5,5'-tetramethylbenzidine (TMB). In some embodiments, the chemiluminescent label may be alkaline phosphatase, and the reagent that causes the chemiluminescent label to emit light may be 4-nitrophenyl phosphate disodium salt hexahydrate (pNPP). In some embodiments, the antibody or the antigen-binding portion thereof of the present application comprises a heavy chain constant region (e.g., an Fc region), and the composition further comprises an antibody that specifically binds to the heavy chain constant region (e.g., the Fc region) and comprises a detectable substance. In some embodiments, the antibody or the antigen-binding portion thereof of the present application comprises a heavy chain constant region (e.g., an Fc region), and the composition further comprises an antibody that specifically binds to the heavy chain constant region (e.g., the Fc region) and comprises a fluorescent label. The fluorescent label may be fluorescein isothiocyanate. In some embodiments, the antibody or the antigen-binding portion thereof of the present application comprises a heavy chain constant region (e.g., an Fc region), the composition further comprises an antibody that specifically binds to the heavy chain constant region (e.g., the Fc region) and comprises a chemiluminescent label, and the composition further comprises a reagent that causes the chemiluminescent label to emit light.

The antibody or the antigen-binding portion thereof of the present application, and the composition of the present application, have a variety of applications, for example, the use in detecting or quantifying a GS linker-containing protein, particularly the use in detecting the presence or amount of the GS linker-containing protein in a sample by, e.g., Western Blot; detecting the presence or amount of cells bound by the GS linker-containing protein in a sample by, e.g., flow cytometry; detecting the presence or amount of cells expressing the GS linker-containing protein in a sample by, e.g., flow cytometry; or detecting the presence or amount of cells expressing the GS linker-containing protein following viral transduction in a sample by, e.g., flow cytometry.

Specifically, the present application provides a method for detecting a GS linker-containing molecule in a sample, the method comprising:
i) contacting the sample with the antibody or the antigen-binding portion thereof of the present application, and
ii) detecting the presence of the antibody or the antigen-binding portion thereof in the sample,
wherein the presence of the antibody or the antigen-binding portion thereof in the sample indicates the presence of a GS linker-containing molecule in the sample.

Alternatively, the present application provides a method for quantifying a GS linker-containing molecule in a sample, the method comprising:
i) contacting the sample with the antibody or the antigen-binding portion thereof of the present application, and
ii) detecting the presence of the antibody or the antigen-binding portion thereof in the sample,
wherein the GS linker-containing molecule in the sample is quantified on the basis of an amount of the antibody or the antigen-binding portion thereof in the sample.

Alternatively, the present application provides a method for purifying a GS linker-containing molecule from a sample, comprising:
i) contacting the sample with a solid support conjugated to the antibody or the antigen-binding portion thereof of the present application,
ii) separating from the sample the sample molecule bound to the solid support in step i) in the sample, and
iii) eluting from the solid support the sample molecule bound to the solid support,
wherein the solid support includes magnetic beads, resin, and agarose beads; and the sample molecule bound to the solid support is a GS linker-containing molecule.

The GS linker that can be detected by the method of the present application may be any GS linker, particularly a GS linker with a length of 6 or more amino acids, particularly a GS linker with a length of 6-20 amino acids, and particularly a GS linker with a length of 6-15 amino acids, including, but not limited to, (G₂S)₂, (G₂S)₄, (G₃S)₃, (G₄S)₂, and (G₄S)₃.

The GS linker-containing molecule may be any GS linker-containing molecule, e.g., a recombinant protein, a nucleic acid-protein complex, etc., e.g., a GS linker-containing antibody or a GS linker-containing chimeric antigen receptor. The GS linker-containing antibody may be a monospecific antibody, a bispecific antibody, or a multispecific antibody, and may comprise various forms of antibodies, such as an scFv, a Fab, and a nanobody.

The sample may be any sample comprising a GS linker-containing molecule, e.g., a sample comprising a GS linker-containing antibody or a sample comprising a GS linker-containing CAR. In some embodiments, the sample may comprise cells treated with a GS linker-containing antibody. In some embodiments, the sample may comprise cells into which a vector has been introduced, wherein the vector comprises a sequence for expressing a GS linker-containing chimeric antigen receptor.

The antibody or the antigen-binding portion thereof of the present application exhibits different binding preferences for various GS linkers. Therefore, if the specific information of the GS linker in the molecule is known prior to detection, the method may further comprise, prior to step i), a step of selecting an antibody or an antigen-binding portion thereof on the basis of the GS linker. If the type of GS linker present in the molecule is unknown prior to detection, multiple GS antibodies or antigen-binding portions thereof in the present application may be used, or an antibody applicable to multiple GS linkers may be selected, for example, an antibody or an antigen-binding portion thereof, wherein the VH CDR1, VH CDR2, VH CDR3, VL CDR1, VL CDR2, and VL CDR3 comprise amino acid sequences set forth in NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively.

The qualitative or quantitative detection of the antibody or the antigen-binding portion thereof may rely on a detectable substance conjugated to the antibody or the antigen-binding portion thereof, or on a secondary antibody capable of binding to the antibody or the antigen-binding portion thereof, all of which are within the ability of those skilled in the art.

The composition of the present application may be a pharmaceutical composition, further comprising a pharmaceutically acceptable carrier. The composition may optionally comprise one or more other pharmaceutically active ingredients, e.g., another anti-tumour antibody or immune-enhancing antibody, or a non-antibody anti-tumour agent or immune-enhancing agent. The composition of the present application can be used in combination with, for example, another anticancer agent or another immune-enhancing agent.

In some embodiments, the composition of the present application may comprise: i) the bispecific molecule of the present application, and ii) a T cell expressing a chimeric antigen receptor, wherein the chimeric antigen receptor comprises: a) an extracellular domain comprising a GS linker, b) a transmembrane domain, and c) an intracellular signalling domain. The chimeric antigen receptor may comprise the GS linker at any suitable position in the extracellular domain so as to activate the T cell upon binding to the bispecific molecule of the present application. In some embodiments, the extracellular domain of the chimeric antigen receptor may comprise a non-antigen-binding single-chain antibody that comprises a GS linker. In some embodiments, the extracellular domain of the chimeric antigen receptor may comprise a non-antigen-binding single-chain antibody, wherein the non-antigen-binding single-chain antibody may comprise a GS linker between the heavy chain variable region-like domain and light chain variable region-like domain thereof.

In some embodiments, the composition of the present application may comprise: i) a T cell expressing the chimeric antigen receptor of the present application, and ii) an antibody or an antigen-binding portion thereof that is linked to a GS linker and targets a disease-associated antigen. The GS linker may be linked to the antibody or the antigen-binding portion thereof that targets the disease-associated antigen via a peptide, e.g., a peptide of 10 to 30 amino acids, such that the binding of the CAR-T to the GS linker does not interfere with the specific binding of the antibody or the antigen-binding portion thereof targeting the disease-associated antigen to the disease-associated antigen. Importantly, the antibody or the antigen-binding portion thereof targeting the disease-associated antigen does not comprise a GS linker, so as to avoid adverse effects on the binding thereof to the disease-associated antigen caused by CAR-T binding.

The above composition can be used for the treatment of various diseases. The diseases may be associated with a disease-associated antigen targeted by a bispecific molecule in a composition, or by an antibody or an antigen-binding portion thereof that comprises a GS linker and targets the disease-associated antigen. The disease-associated antigen may be a tumour-associated antigen, etc.

The composition may comprise any number of excipients. Excipients that can be used include carriers, surfactants, thickening or emulsifying agents, solid binders, dispersing or suspending agents, solubilizers, colorants, flavouring agents, coatings, disintegrants, lubricants, sweeteners, preservatives, isotonic agents, and combinations thereof. The selection and use of suitable excipients is taught in Gennaro, ed., Remington: *The Science and Practice of Pharmacy*, 20th Ed. (Lippincott Williams & Wilkins 2003).

Preferably, the composition is suitable for intravenous, intramuscular, subcutaneous, parenteral, spinal, or epidermal administration (e.g., via injection or bolus injection). Depending on the route of administration, the active ingredient can be encapsulated in a material to protect it from acids and other natural conditions that may inactivate it. "Parenteral administration" refers to administration other than enteral and topical administration, and is generally performed via injection, including but not limited to intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural and intrasternal injection and bolus injection. Alternatively, the antibody of the present application can be administered via non-parenteral routes, e.g., external, epidermal, or mucosal administration, e.g., intranasal, oral, vaginal, rectal, sublingual, or topical administration.

The compositions may be in the form of sterile aqueous solutions or dispersions. They may also be formulated in microemulsions, liposomes, or other ordered structures suitable for high concentrations of drugs.

The amount of active ingredient that is prepared together with a carrier material into a single dosage form will vary depending on a subject treated and a specific mode of administration, and is essentially the amount of the composition that produces a therapeutic effect. The amount of the active ingredient in combination with a pharmaceutically acceptable carrier is, by percentage, about 0.01% to about 99%.

The administration regimen is adjusted to provide the optimal desired response (e.g., a therapeutic response). For example, a bolus injection can be administered, multiple divided doses can be administered over time, or the dose can be reduced or increased in proportion to the severity of the condition being treated. It is particularly advantageous to formulate parenteral compositions in dosage unit forms for ease of administration and uniformity of dosage. Dosage unit form refers to physically discrete units suitable for single administration to a subject treated; each unit contains a predetermined amount of the active ingredient calculated to produce the desired therapeutic effect in association with the pharmaceutical carrier. Alternatively, the antibody can be administered as a sustained-release formulation, in which case the required frequency of administration is reduced.

For the administration of the antibody, the dosage may be about 0.001 to 100 mg/kg of host body weight. An exemplary therapeutic regimen involves administration once weekly.

A "therapeutically effective amount" of the composition of the present application results in a reduction in the severity of disease symptoms and an increase in the frequency and duration of asymptomatic periods. For example, for the treatment of a subject with a tumour, a "therapeutically effective amount" inhibits tumour growth by at least about 20%, at least about 40%, even at least about 60%, and more particularly at least about 80%, as compared to an untreated subject. A therapeutically effective amount of a therapeutic antibody may reduce tumour size, or alleviate a symptom in a subject, which may be a human or another mammal.

The compositions may be sustained-release agents, including implants and microencapsulated delivery systems. Biodegradable and biocompatible polymers such as ethylene-vinyl acetate, polyanhydrides, polyglycolic acid, collagen, polyorthoesters, and polylactic acid can be used. The compositions can be administered by a medical device, such as (1) a needleless hypodermic injection device (e.g., U.S. Pat. Nos. 5,399,163; 5,383,851; 5,312,335; 5,064,413; 4,941,880; 4,790,824; and 4,596,556); (2) a micro-infusion pump (U.S. Pat. No. 4,487,603); (3) a transdermal administration device (U.S. Pat. No. 4,486,194); (4) a bolus injection device (U.S. Pat. Nos. 4,447,233 and 4,447,224); and (5) an osmotic device (U.S. Pat. Nos. 4,439,196 and 4,475,196).

In certain embodiments, the components of the composition of the present application can be formulated to ensure appropriate *in vivo* distribution. For example, to ensure that the therapeutic components in the composition of the present application cross the blood-brain barrier, the components can be formulated in liposomes, which may additionally comprise targeting functional moieties to enhance the selective delivery to specific cells or organs.

Various aspects and embodiments of the present application will be discussed with reference to the accompanying drawings and the following examples. Other aspects and embodiments will be apparent to those skilled in the art. All documents described herein are incorporated herein by reference in their entireties. Although the present application has been described in conjunction with exemplary embodiments, many equivalent modifications and variations will be apparent to those skilled in the art from the present application. Accordingly, the exemplary embodiments of the present application are intended to be illustrative and not restrictive. Various modifications can be made to the embodiments without departing from the spirit and scope of the present application.

### Example 1. Animal immunization and serum titre determination

The (G₄S)₃ polypeptide (SEQ ID NO: 68) was added to maleimide-activated bovine serum albumin (BSA) carrier protein at a molar ratio of 35 : 1, and reacted at room temperature for 2 h to conjugate the (G₄S)₃ polypeptide to BSA. Subsequently, (G₄S)₃-BSA was used as an antigen for animal immunization. Specifically, three New Zealand rabbits numbered R13087#, R13088#, and R13089# were immunized subcutaneously with an emulsion containing 200 µg of (G₄S)₃-BSA and 200 µl of complete Freund's adjuvant (Sigma-Aldrich, CAT#: F5881). The New Zealand rabbits were subjected to booster immunization by subcutaneous injection of an emulsion containing 200 µg of (G₄S)₃-BSA and incomplete Freund's adjuvant (Sigma-Aldrich, CAT#: F5506) every two weeks for a total of four times.

Four days before blood collection, serum titres of the animals were determined using an indirect ELISA assay. Specifically, the (G₄S)₃ polypeptide was added to maleimide-activated ovalbumin (OVA) carrier protein at a molar ratio of 35 : 1, and reacted at room temperature for 2 h to conjugate the (G₄S)₃ polypeptide to OVA. Then, 1 µg/ml (G₄S)₃ polypeptide or 1 µg/ml (G4S)3-OVA was added to a microplate at 100 µl/well, followed by coating overnight at 4°C. After washing the microplate once with PBST (PBS containing 0.05% (v/v) Tween), PBST containing 1% BSA (w/v) was added at 200 µl/well, and the plate was blocked at 37°C for 2 hours. After that, the blocking solution was discarded. 100 µl of serially diluted animal serum was added to each well (the first well was diluted at a ratio of 1 : 1000 with PBST containing 1% BSA, followed by two-fold serial dilutions), and the plate was incubated at 37°C for 1 hour. After washing the microplate four times with PBST, 100 µl/well of HRP-conjugated anti-rabbit IgG Fc secondary antibody (GenScript, CAT#: A01856, diluted at a ratio of 1 : 20,000 in PBST containing 1% BSA) was added, and the plate was incubated at 37°C for 0.5 hours. The microplate was washed four times with PBST. Then, TMB substrate solution was added, and the plate was incubated at room temperature in the dark for 15 minutes. Finally, 50 µl of 0.5 M HCl stop solution was added to terminate the reaction. The plate was read at 450 nm using a microplate reader. The criterion for determining a valid serum titre was that the OD₄₅₀ value of the diluted serum was greater than 2.1 times the OD₄₅₀ value of the blank background.

The results showed that the titres of sera from R13087# and R13088# against the (G₄S)₃ polypeptide were higher than that from R13089#, with a titre of 1 : 512,000; the titres of sera from R13087# and R13088# against (G₄S)₃-OVA were also higher than that from R13089#, with titres greater than 1 : 512,000, indicating the presence of antibodies specific to (G₄S)₃, including (G₄S)₃ in (G₄S)₃-OVA, in the animal sera. In summary, R13087# and R13088# exhibited higher serum titres against both the (G₄S)₃ peptide and (G₄S)₃-OVA than R13089#. Therefore, these two animals were selected for further booster immunization via intraperitoneal and intravenous injection of 400 µg of (G₄S)₃-BSA (without adjuvant).

### Example 2. Acquisition of single B cells expressing antibodies

After the booster immunization was completed, fresh blood was collected from the rabbits and subjected to isolation within 4 hours after removal from the body. Peripheral blood mononuclear cells (PBMCs) were isolated using Ficoll^{®}. Human peripheral blood lymphocyte separation medium (prepared with FICOLL) (Solarbio, CAT#: P8900) was added to centrifuge tubes, and blood was added at a ratio of 1 : 1 carefully and slowly to prevent mixing of the blood and the separation medium. After that, the centrifuge was pre-cooled to 25°C, and centrifugation was performed at 400 × g for 30 minutes. After centrifugation, the blood separation status in the tubes was observed, and white PBMCs floating in the turbid layer were aspirated. Subsequently, the PBMCs were added to a cell culture plate pre-coated with 1 mg of (G₄S)₃-OVA to enrich for memory B cells specific for (G₄S)₃. The number of enriched cells was counted using a hemocytometer, and the cells were serially diluted until the cell density reached 5-15 cells/ml. 200 µl of the cell suspension was transferred into a 96-well plate using a pipette, at a density of 1-3 cells/well. After culturing and expanding the single B cells, positive clones recognizing (G₄S)₃ were selected and further cultured in a 24-well plate.

Indirect ELISA was used to detect the binding ability of antibodies secreted by B cells to proteins containing (G₄S)₃. Specifically, 1 µg/mL (G₄S)₃-OVA was added to a microplate at 100 µl/well, followed by coating overnight at 4°C. After washing the microplate once with PBST (containing 0.05% (v/v) Tween), PBST containing 1% BSA (w/v) was added at 200 µl/well, and the plate was blocked at 37°C for 2 hours. Subsequently, the blocking solution was discarded. 100 µl of B cell culture supernatant was added to each well, and the plate was incubated at 37°C for 1 hour. The above positive serum (rabbit serum after booster immunization) diluted 1000-fold was used as a positive control, and blank medium was used as a negative control. After washing the microplate four times with PBST, 100 µl/well of HRP-conjugated anti-rabbit IgG Fc secondary antibody (GenScript, CAT#: A01856, diluted at a ratio of 1 : 20,000 in PBST containing 1% BSA) was added, and the plate was incubated at 37°C for 0.5 hour. The microplate was washed four times with PBST. Then, TMB substrate solution was added, and the plate was incubated at room temperature in the dark for 15 minutes. Finally, 50 µl of 0.5 M HCl stop solution was added to terminate the reaction. The plate was read at 450 nm using a microplate reader.

The results showed that the OD₄₅₀ value of the positive control was 2.784, and the OD₄₅₀ value of the negative control was 0.073. A total of eight positive clones were detected by indirect ELISA, namely R177.B8, R177.B11, R177.D13, R177.E4, R177.G3, R177.G5, R177.G12, and R177.I8, with OD₄₅₀ readings of 2.55, 0.899, 2.539, 1.668, 0.8, 1.005, 2.447, and 1.118, respectively.

### Example 3. Sequencing of antibody variable regions and preparation of recombinant antibodies

Total RNA was extracted from B cells using TRIzol (Ambion, CAT#: 15596-026) and reverse-transcribed into cDNA using antibody isotype-specific primers and universal primers (Takara PrimeScript^{™} first-strand cDNA synthesis kit, CAT#: 6110A). Subsequently, the heavy and light chain variable region fragments of rabbit immunoglobulin were amplified by RACE PCR, and the resulting PCR fragments were subcloned into the pMD18-T vector system (Takara, CAT#: 6011). Insert fragments were sequenced using vector-specific primers. The nucleotide and protein sequences of the heavy and light chain variable regions of these eight monoclonal antibodies were finally obtained and are listed in Table 1 and Table 3.

**Table 1. Sequences or SEQ ID NOs of variable regions and CDRs of the antibodies of the present application**

| mAb ID | Variable region and CDR sequences |
|---|---|
| R177.B8 | Heavy chain variable region (VH CDR1, VH CDR2, VH CDR3 and VH sequentially from top to bottom) |
| | NYAIM (SEQ ID NO: 1) |
| | VIYANGDPYCASWAKG (SEQ ID NO: 5) |
| | GGF QSLEESGGRLVTPGGSLTLTCTVSGIDLSNYAIMWVRQAPGKGLEWIG VIYANGDPYCASWAKGRFTISKTSSTTVDLKMTSLTTEDTATYFCAR GGFWGPGTLVTVSS (SEQ ID NO: 32) |
| | Light chain variable region (VL CDR1, VL CDR2, VL CDR3 and VL sequentially from top to bottom) |
| | QSSQSIYNKNDLV (SEQ ID NO: 14) |
| | GISTLDS (SEQ ID NO: 20) |
| | LGGFSCSSGDCGA (SEQ ID NO: 25) |
| | QVLTQTPSPVSAAVGGTVTINCQSSQSIYNKNDLVWLQQKPGQPPKR LIYGISTLDSGVSSRFKGSGSGTQFTLTISGVQCDDAATYYCLGGFSCS SGDCGAFGGGTEVVVR (SEQ ID NO: 40) |
| R177.B11 | Heavy chain variable region (VH CDR1, VH CDR2, VH CDR3 and VH sequentially from top to bottom) |
| | NYDMY (SEQ ID NO: 2) |
| | VIYVNGNTHYASWAKG (SEQ ID NO: 6) |
| | GPF QSLEESGGRLVTPGGSLTLTCTASGIDLRNYDMYWARQALGKGLEW VGVIYVNGNTHYASWAKGRFTISRTSSTTVDLKMTSLTTEDTATYFC VRGPFWGPGTLVTVSS (SEQ ID NO: 33) |
| | Light chain variable region (VL CDR1, VL CDR2, VL CDR3 and VL sequentially from top to bottom) |
| | QASQSVWKNKDLV (SEQ ID NO: 15) |
| | DASTLSS (SEQ ID NO: 21) |
| | LGGFSCSRGDCGA (SEQ ID NO: 26) |
| | AQVLTQTPSPVSAAVGGTVTINCQASQSVWKNKDLVWYQQKAGQPP KRLIYDASTLSSGVSSRFKGSGSGTQFSLTMTDVQCDDAATYYCLGG FSCSRGDCGAFGGGTEGGGQ (SEQ ID NO: 41) |
| R177.E4 | Heavy chain variable region (VH CDR1, VH CDR2, VH CDR3 and VH sequentially from top to bottom) |
| | NYDMY (SEQ ID NO: 2) |
| | VSYKNGRAHYASWAKG (SEQ ID NO: 8) |
| | GPL QSLEESGGRLVTPGGSLTLTCTASGIDLKNYDMYWARQAPGKGLDWI GVSYKNGRAHYASWAKGRFTISKTSTTVDLKMTSLTTEDTATYFCVR GPLCGPGTLVTVSS (SEQ ID NO: 35) |
| | Light chain variable region (VL CDR1, VL CDR2, VL CDR3 and VL sequentially from top to bottom) |
| | QASQSVWNNNDLV (SEQ ID NO: 17) |
| | DASTLSS (SEQ ID NO: 21) |
| | LGGFSCSTGDCGA (SEQ ID NO: 28) |
| | AQVLTQTPSPVSAAVGGTVTINCQASQSVWNNNDLVWYQQKPGQRP KRLIYDASTLSSGVSSRFKGSGSGTQFTLTISGVQCDDAATYYCLGGF SCSTGDCGAFGGGTEVVVK (SEQ ID NO: 43) |
| R177.G3 | Heavy chain variable region (VH CDR1, VH CDR2, VH CDR3 and VH sequentially from top to bottom) |
| | NYDMY (SEQ ID NO: 2) |
| | CIYTGSGGKRYANWAKG (SEQ ID NO: 9) |
| | GPF QSLEESGGRLVTPGGSLTLTCTASGIDLNNYDMYWARQAPGKGLEWI GCIYTGSGGKRYANWAKGRFTFSRTSTTVDLKMTSLTTEDTATYFCV RGPFWGPGTLVTVSS (SEQ ID NO: 36) |
| | Light chain variable region (VL CDR1, VL CDR2, VL CDR3 and VL sequentially from top to bottom) |
| | QASQSVWNNNDLV (SEQ ID NO: 17) |
| | DASILSS (SEQ ID NO: 23) |
| | LGGFSCRSGDCGA (SEQ ID NO: 29) |
| | AQVLTQTPSPVSAAVGGTVTIICQASQSVWNNNDLVWYQQKAGQR PKRLIYDASILSSGVSSRFKGSGSGTQFSLTVSDVQCDDAATYYCLGG FSCRSGDCGAFGGGTEVVVK (SEQ ID NO: 44) |
| R177.G5 | Heavy chain variable region (VH CDR1, VH CDR2, VH CDR3 and VH sequentially from top to bottom) |
| | NYDMY (SEQ ID NO: 2) |
| | VSYASGRTYSVRWAKG (SEQ ID NO: 10) |
| | GPL QSLEESGGRLVTPGGSLTLTCTASGIDLNNYDMYWARQAPGKGLDWI GVSYASGRTYSVRWAKGRFTISKTSSTTVDLKMTSLTTEDTATYFCV RGPLWGPGTLVTVSS (SEQ ID NO: 37) |
| | Light chain variable region (VL CDR1, VL CDR2, VL CDR3 and VL sequentially from top to bottom) |
| | QASQTVWKNNDLV (SEQ ID NO: 18) |
| | DASTLSS (SEQ ID NO: 21) |
| | LGGFSCSSGDCGA (SEQ ID NO: 25) |
| | AQVLTQTPSPVSAAVGGTVTINCQASQTVWKNNDLVWYQQKPGQRP KRLIYDASTLSSGVSSRFKGSGSGTQFSLTMSDVQCDDAATYYCLGGF SCSSGDCGAFGGGTEVVVK (SEQ ID NO: 45) |
| R177.I8 | Heavy chain variable region (VH CDR1, VH CDR2, VH CDR3 and VH sequentially from top to bottom) |
| | NYDMY (SEQ ID NO: 2) |
| | VIYKNGNAHSASWARG (SEQ ID NO: 12) |
| | GPF QSLEESGGRLVTPGGFLTLTCIASGIDLRNYDMYWARQALGKGLEWV GVIYKNGNAHSASWARGRFTISRTSSTTVDLKMTSLTTEDTATYFCVR GPFWGPGTLVTVSS (SEQ ID NO: 39) |
| | Light chain variable region (VL CDR1, VL CDR2, VL CDR3 and VL sequentially from top to bottom) |
| | QASQSVWNNNDLV (SEQ ID NO: 17) |
| | DASTLSS (SEQ ID NO: 21) |
| | LGGFSCSRGDCGS (SEQ ID NO: 31) |
| | AQVLTQTPSPVSAAVGGTVTINCQASQSVWNNNDLVWYQQKPGQP PKRLIYDASTLSSGVSSRFRGSGSGTQFSLTMTDVQCDDAATYYCLGG FSCSRGDCGSFGGGTEVVVK (SEQ ID NO: 47) |
| R177.D13 | Heavy chain variable region (VH CDR1, VH CDR2, VH CDR3 and VH sequentially from top to bottom) |
| | NHAIM (SEQ ID NO: 3) |
| | VIYSNGNPYCARWVKG (SEQ ID NO: 7) |
| | GGF QSLEESGGRLITPGGSLTLTCTVSGIDLSNHAIMWVRQAPGKGLEWIG VIYSNGNPYCARWVKGRFTISKTSSTTVDLKMTSLTTEDTATYFCARG GFWGPGTLVTVSS (SEQ ID NO: 34) |
| | Light chain variable region (VL CDR1, VL CDR2, VL CDR3 and VL sequentially from top to bottom) |
| | KTSQSIYNNNDLV (SEQ ID NO: 16) |
| | GVSTLDS (SEQ ID NO: 22) |
| | LGGFRCSSGDCGA (SEQ ID NO: 27) |
| | AQVLTQTLSPVSAAVGGTVTINCKTSQSIYNNNDLVWLQQKPGQP PKRLIYGVSTLDSGVSSRFKGSGSGTQFTLTISGMQCEDAATYYCLGG FRCSSGDCGASGGGTEVVVR (SEQ ID NO: 42) |
| R177.G12 | Heavy chain variable region (VH CDR1, VH CDR2, VH CDR3 and VH sequentially from top to bottom) |
| | TNAMT (SEQ ID NO: 4) |
| | TITISGNKYYASWAKG (SEQ ID NO: 11) |
| | GVVQSLVL (SEQ ID NO: 13) |
| | QSVEESGGRLVTPGTPLTLTCTVSGFSLSTNAMTWVRQAPGEGLEWI GTITISGNKYYASWAKGRFTISRTSTTVDLKMTSLTAADTATYFCARG VVQSLVLWGQGTLVSVSS (SEQ ID NO: 38) |
| | Light chain variable region (VL CDR1, VL CDR2, VL CDR3 and VL sequentially from top to bottom) |
| | RSSQNVYNNNGLG (SEQ ID NO: 19) |
| | DAADLAS (SEQ ID NO: 24) |
| | AGGYSSGSIDNT (SEQ ID NO: 30) |
| | AVVMTQTPASVSAAVGGTVTISCRSSQNVYNNNGLGWYQQKPGQP PKLLIYDAADLASGVPSRFKGSGSGTQFTLTISDLECDDAATYYCAGG YSSGSIDNTFGGGTEVVVK (SEQ ID NO: 46) |

DNA fragments encoding the light chain variable region (with the nucleotide sequence set forth in SEQ ID NO: 56, 57, 58, 59, 60, 61, 62, or 63) and the light chain constant region (with the nucleotide sequence set forth in SEQ ID NO: 67), and the heavy chain variable region (with the nucleotide sequence set forth in SEQ ID NO: 48, 49, 50, 51, 52, 53, 54, or 55) and the heavy chain constant region (with the nucleotide sequence set forth in SEQ ID NO: 65) were synthesized and cloned into the pTT5 expression vector. After the correct sequence was confirmed by sequencing, the above plasmids were co-transfected into CHO-S cells. After the transfection was completed, the cells were cultured in a shake flask at 37°C for 6 days, and the supernatant was collected for antibody purification. In antibody purification, the tubing and Protein A column were first washed with 0.2 M NaOH to remove pyrogens. The column was then equilibrated with equilibration buffer (50 mM Tris, 150 mM NaCl, pH 8.0). The harvested cell supernatant was diluted at a ratio of 1 : 1 with 2× equilibration buffer and filtered through a 0.22 µm filter to remove insoluble particles, bacteria, etc. After passing the cell supernatant onto the Protein A column, the column was washed with 1× equilibration buffer to remove non-specifically bound proteins. Subsequently, the target antibody was eluted with 0.1 M sodium citrate (pH 3.5) that had been filter-sterilized using a 0.22 µm filter. One-ninth volume of sterile 1 M Tris-HCl (pH 9.0) was added to the eluate containing the target antibody for neutralization, resulting in a final pH of 7.4. Subsequently, the antibody was concentrated by ultrafiltration using a 50 KD ultrafiltration membrane, and the elution buffer was exchanged to PBS (pH 7.2). Subsequently, the antibody concentration was determined using a Nanodrop spectrophotometer, with an extinction coefficient of 1.43. Finally, the molecular weight and purity of the purified antibodies were analysed by reducing and non-reducing polyacrylamide gel electrophoresis (SDS-PAGE).

The identification results of the molecular weight and purity of the eight antibodies showed that, under non-reducing conditions, the molecular weight of each antibody was slightly lower than the actual molecular weight, at about 120 kDa. Under reducing conditions, as the reducing agent disrupted the disulfide bond structures, the heavy and light chains of the antibody molecules were visualized on the gel, with molecular weights of about 55 kDa and 25 kDa, respectively. The purity of all eight antibodies, as determined by reducing electrophoresis, was greater than 90%.

### Example 4. Binding affinity of antibodies for different GS linkers

The binding ability of the purified antibodies to various GS linkers was evaluated using an indirect ELISA method.

Specifically, different GS linkers, namely (G₂S)₂ (SEQ ID NO: 69), (G₂S)₄ (SEQ ID NO: 70), (G₃S)₃ (SEQ ID NO: 71), G₄S (SEQ ID NO: 72), (G₄S)₂ (SEQ ID NO: 73), and (G₄S)₃ (SEQ ID NO: 68), were each prepared at a concentration of 4 µg/mL and added to a microplate at 100 µl/well, followed by coating overnight at 4°C. After washing the microplate once with PBST (PBS containing 0.05% (v/v) Tween), PBST containing 1% BSA (w/v) was added at 200 µl/well, and the plate was blocked at 37°C for 2 hours. Subsequently, the blocking solution was discarded. 100 µl of serially diluted antibodies of the present application were added to each well, and then the plate was incubated at 37°C for 1 hour. After washing the microplate four times with PBST, 100 µl/well of HRP-conjugated anti-rabbit IgG Fc secondary antibody (GenScript, CAT#: A01856, diluted at a ratio of 1 : 20,000 in PBST containing 1% BSA) was added, and the plate was incubated at 37°C for 0.5 hour. The microplate was washed four times with PBST. Then, TMB substrate solution was added, and the plate was incubated at room temperature in the dark for 15 minutes. Finally, 50 µl of 0.5 M HCl stop solution was added to terminate the reaction. The plate was read at 450 nm using a microplate reader.

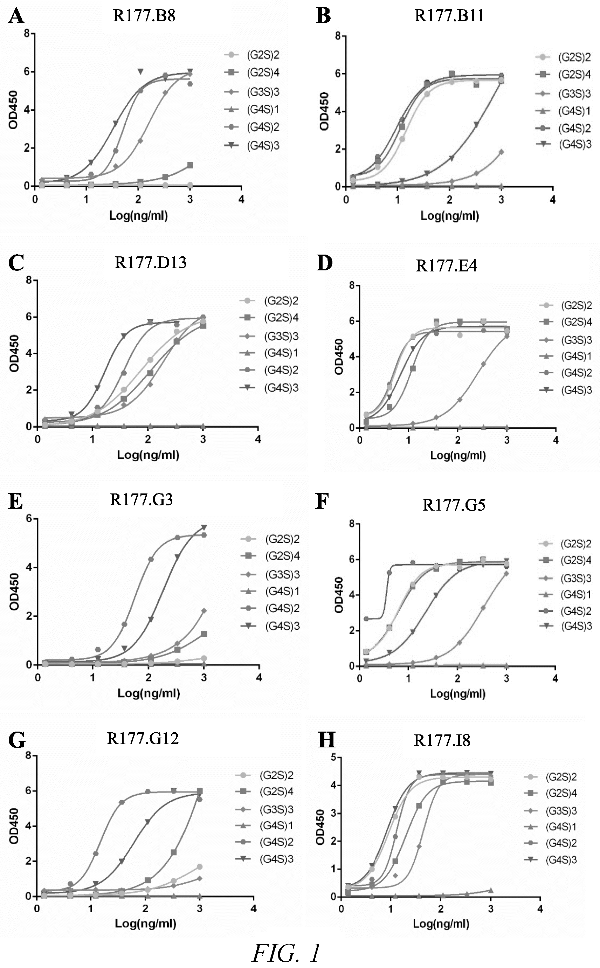

As shown in FIG. 1 (A-H), the eight antibodies of the present application exhibited weak binding to the (G₄S)₁ linker, strong binding to (G₄S)₃, and varying binding affinities for the other linkers. Among them, antibody R177.B8 exhibited strong binding to (G₃S)₃, (G₄S)₂, and (G₄S)₃; antibody R177.B11 exhibited strong binding to (G₂S)₂, (G₂S)₄, (G₄S)₂, and (G₄S)₃; R177.G3 exhibited strong binding to (G₄S)₂ and (G₄S)₃; and R177.G12 showed strong binding to (G₂S)₄, (G₄S)₂, and (G₄S)₃. Except for the (G₄S)₁ linker, R177.D13, R177.E4, R177.G5, and R177.I8 all had good binding effects on all other tested GS linkers.

### Example 5. Determination of binding affinity of antibodies for GS linker-containing scFv

The binding ability of the eight antibodies of the present application to an scFv containing a (G₄S)₃ linker was detected using the Gator label-free bioanalysis system. The linker-containing scFv was prepared based on the sequence of brolucizumab, which specifically binds to VEGFA, and comprises, from the N-terminus to the C-terminus, a heavy chain variable region, a linker, and a light chain variable region. The amino acid sequences of the heavy chain variable region and the light chain variable region are set forth in SEQ ID NO: 74 and SEQ ID NO: 75, respectively.

A protein A probe was selected and soaked in 250 µl of buffer K (1× PBS + 0.002% Tween 20 + 0.02% BSA) for 10 min before use. Subsequently, the antibodies of the present application were diluted with Buffer K to a final concentration of 5.0 µg/ml, and 200 µl of the dilution was added to each well. The scFv was diluted with Buffer K at an initial concentration of 5.0 µg/ml by 2-fold serial dilution to obtain a total of three concentration points. Each gradient sample had a volume of 200 µl and was added to the plate. A 10 mM glycine-hydrochloric acid buffer (pH 2.0) was used as the regeneration buffer, and buffer K was used as the neutralization buffer. The instrument lid was opened, the gasket was lifted to tilt shaker A, and the sample plate was placed into position. The probe plate was placed at the position of shaker B, and the lid was closed. The Kinetics K module was selected. Step 1 was set to baseline, with the position selected to be the position of buffer K, and the time set to 120 s; step 2 was set to loading, with the position selected to be the position of the antibodies of the present application, and the time set to 120 s; step 3 was set to baseline, with the position selected to be the position of buffer K, and the time set to 30 s; step 4 was set to association, with the position selected to be the position of scFv, and the time set to 200 s; step 5 was set to dissociation, with the position selected to be the position of buffer K, and the time set to 300 s; the regeneration cycles were set to 3. Subsequently, the program was run to detect the binding affinity of the antibodies of the present application to the GS linker-containing scFv.

The results are shown in Table 2. The affinities of the (G₄S)₃-containing scFv for the eight antibodies, namely R177.B8, R177.B11, R177.D13, R177.E4, R177.G3, R177.G5, R177.G12, and R177.I8, were all at the nmol level, with values of 0.334 nmol, 3.26 nmol, 2.07 nmol, 1.95 nmol, 2.79 nmol, 2.82 nmol, 2.82 nmol, and 2.80 nmol, respectively. This indicates that the eight antibodies of the present application all exhibit high binding affinity for (G₄S)₃-scFv.

**Table 2. Binding affinities of eight antibodies to (G₄S)₃-containing scFv**

| mAb ID | Dissociation constant (1/s) | Association constant (1/Ms) | Binding affinity (M) |
|---|---|---|---|
| R177.B8 | 4.61E-05 | 1.38E+05 | 3.34E-10 |
| R177.B11 | 0.000244 | 7.50E+04 | 3.26E-09 |
| R177.D13 | 0.00021 | 1.01E+05 | 2.07E-09 |
| R177.E4 | 0.000173 | 8.86E+04 | 1.95E-09 |
| R177.G3 | 0.00026 | 9.32E+04 | 2.79E-09 |
| R177.G5 | 0.000275 | 9.76E+04 | 2.82E-09 |
| R177.G12 | 0.000275 | 9.76E+04 | 2.82E-09 |
| R177.I8 | 0.000272 | 9.71E+04 | 2.80E-09 |

### Example 6. Detection of GS linker-containing scFv by antibodies in Western Blot

200 µL of RIPA lysis buffer (Beyotime, CAT#: P0013B) was added to 1 × 10⁶ HEK-293 cells. The mixture was mixed thoroughly and reacted at 4°C for 30 min. Subsequently, centrifugation was performed at 12,000 × g for 15 min, and the supernatant was collected. The protein concentration of the supernatant was determined using a BCA kit (Thermo, CAT#: 23225) according to the operation method in the instructions. The supernatant was diluted with RIPA lysis buffer to a protein concentration of 2 mg/mL.

The scFv containing (G₄S)₃ used in Example 5 was diluted to 20 µg/mL with PBS. The obtained diluted supernatant of HEK-293 cell lysate was mixed with PBS at a volume ratio of 1 : 1 and designated as sample 1; the obtained scFv solution was mixed with PBS at a volume ratio of 1 : 1 and designated as sample 2; and the obtained diluted supernatant of HEK-293 cell lysate was mixed with the obtained scFv solution at a volume ratio of 1 : 1 and designated as sample 3. One-half volume of 4× loading buffer (GenScript, CAT#: M00676) and one-half volume of PBS were added to samples 1, 2, and 3. The mixtures were mixed thoroughly and incubated in a metal bath at 100°C for 10 minutes. Subsequently, loading was performed at 10 µL/well, followed by polyacrylamide gel electrophoresis. After the electrophoresis was completed, the proteins were transferred onto a PVDF membrane using the eBlot^{™} L1 rapid transfer system. The eight antibodies of the present application at 0.5 µg/mL were used as primary antibodies separately, and incubation was performed at room temperature for 2 hours. Subsequently, the membrane was washed three times with PBST for five minutes each time. HRP-conjugated anti-rabbit IgG Fc secondary antibody (GenScript, CAT#: A01856) at 0.1 µg/mL was added, followed by incubation at room temperature for 1 hour. The membrane was washed three times with PBST for five minutes each time. Finally, exposure was performed using ECL chemiluminescent substrate (TANON, CAT#: 180-5001).

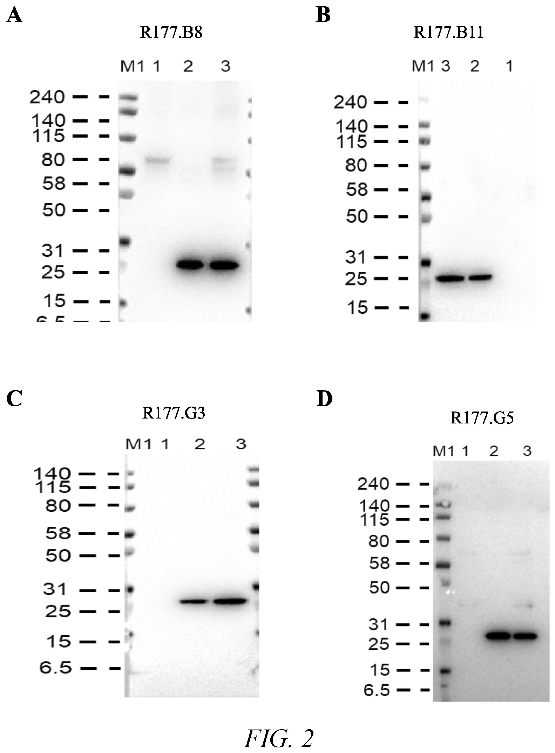

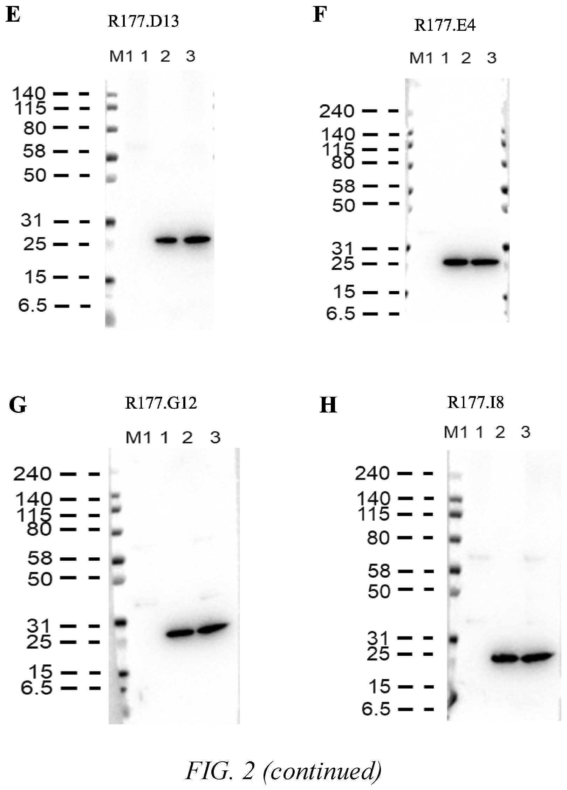

The results are shown in FIG. 2 (A-H). 50 ng of scFv containing the (G₄S)₃ linker was effectively detected by all eight antibodies of the present application, indicating that the antibodies of the present application exhibited high detection sensitivity. In addition, no obvious non-specific bands were observed in the HEK-293 cell lysate supernatant, indicating the high binding specificity of the antibodies of the present application.

### Example 7. Detection of GS linker-containing protein by antibodies in flow cytometry

The sequences of rituximab targeting CD20 and muromonab targeting CD3 were selected to construct a bispecific antibody. The specific structure was as follows: rituximab VL-(G4S)3 linker-VH-(G4S) linker-muromonab VH-(G4S)3 linker-VL-His tag. The amino acid sequences of the heavy chain and light chain variable regions of rituximab and the heavy chain and light chain variable regions of muromonab are set forth in SEQ ID NOs: 76, 77, 78, and 79, respectively.

5 × 10⁵ Raji cells were taken, washed once with 500 µl of FACS buffer (PBS + 1% BSA), and centrifuged at 1000 rpm for 5 minutes. The supernatant was then discarded. The cells were resuspended in 200 µl of FACS buffer, and 2 µg of the above bispecific antibody was added. The mixture was incubated at room temperature for 20 min. Subsequently, the cells were washed once with 500 µl of FACS buffer and centrifuged at 1000 rpm for 5 minutes. The supernatant was then discarded. The cells were resuspended in 200 µl of FACS buffer, and 2 µg of the antibody R177.B8, R177.D13 or R177.G12 of the present application, or FITC-labelled mouse anti-His positive control antibody (GenScript, CAT#: A01620) was added, followed by incubation at room temperature for 20 minutes. The cells were washed once with 500 µl of FACS buffer and centrifuged at 1000 rpm for 5 minutes. The supernatant was then discarded. The cells were resuspended in 200 µl of FACS buffer. 2 µg of FITC-labelled anti-rabbit IgG Fc secondary antibody (Jackson, CAT#: 111-095-008) was added to the cells incubated with the antibodies of the present application, followed by reaction at room temperature for 20 minutes. The percentage of fluorescent cells relative to total cells was detected by flow cytometry.

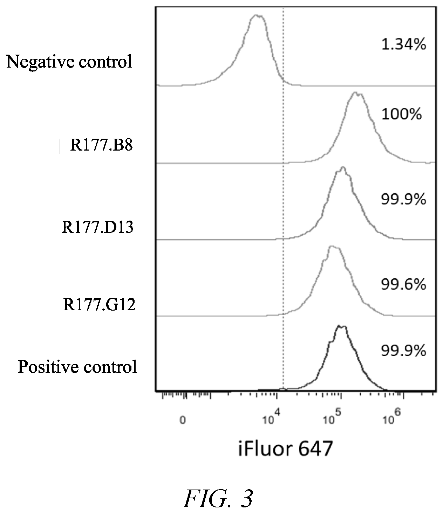

As shown in the results of FIG. 3, the proportions of positive cells detected by the antibodies R177.B8, R177.D13, and R177.G12 of the present application were 100%, 99.9% and 99.6%, respectively, which were consistent with those of the anti-His positive control antibody. It can be seen that the antibodies of the present application can detect GS linker-containing proteins in flow cytometry.

### Example 8. Detection of lentiviral transduction titre by antibodies

Lentiviruses carrying the gene encoding a scFv-containing chimeric antigen receptor (CAR) were serially diluted and used to infect HEK-293 cells to obtain CAR-expressing HEK-293 cells. The scFv contained in the CAR comprised a (G4S)₃ linker between the heavy chain variable region and the light chain variable region.

Specifically, HEK-293 cells were uniformly seeded in a 24-well plate at a cell density of 40,000 cells per well, with 480 µL of medium in each well. The concentrated lentivirus was serially diluted with serum-free DMEM medium at ratios of 1 : 81, 1 : 243, 1 : 729, and 1 : 2187. After thorough mixing by vortexing, 20 µL of each dilution was added to the 24-well plate seeded with HEK-293 cells. After 12 h of infection, the medium was replaced with complete medium, and infection was further performed for 48 h. The cells were washed once with 500 µl of FACS buffer (PBS + 1% BSA) and centrifuged at 1000 rpm for 5 minutes. The supernatant was then discarded. The cells were resuspended in 100 µl of FACS buffer, and 1 µg of FITC-labelled antibody R177.B8, R177.D13 or R177.G12 was added, followed by incubation at room temperature for 20 minutes. The cells were washed once with 500 µl of FACS buffer and centrifuged at 1000 rpm for 5 minutes. The supernatant was then discarded. The cells were resuspended in 100 µl of FACS buffer. The positive rate of CAR (i.e., the percentage of fluorescent cells relative to total cells) was detected by flow cytometry, based on which the viral transduction titre was calculated.

The antibodies were labelled with FITC as follows. FITC (SIGMA-ALDRICH, CAT#: F7250-100MG) was dissolved in DMF to a concentration of 1 mg/mL. Subsequently, FITC was added to the antibody R177.B8, R177.D13 or R177.G12 of the present application at a mass ratio of FITC to antibody of 0.2 : 1, followed by reaction at room temperature for 2 h. After that, the reaction solution was placed in an ultrafiltration tube and centrifuged at 12,000 × g for 10 min at 4°C. After the centrifugation was completed, the liquid in the outer tube was discarded, and the inner tube was replenished with PBS. The centrifugation-PBS replenishment process was repeated three times to replace the antibody-FITC reaction buffer with PBS, thereby completing the FITC labelling of the antibody.

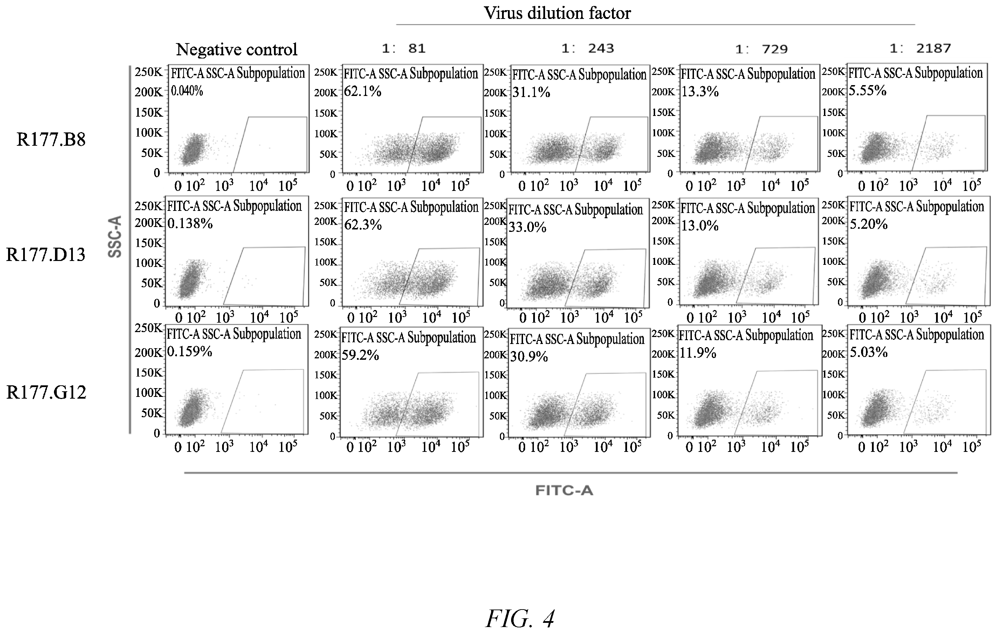

As shown in FIG. 4, after binding and staining of HEK-293 cells using the antibodies of the present application, HEK-293 cells infected with different viral loads all exhibited distinct population resolution. As the viral dilution ratio increased, the proportion of fluorescent cells gradually decreased, which was consistent with the theoretical expectation of a decreased number of infected cells or gradually reduced CAR expression on the cells. When cells were infected with the virus at the same dilution ratio, the CAR positive rates detected by the three antibodies of the present application were basically consistent. According to the titre calculation formula: Viral titre = (CAR-positive cell ratio × initial cell number × viral dilution ratio) / (viral volume). For example, when detected with R177.B8: Viral titre at 1 : 2187 dilution = (5.55% × 40,000 cells × 2187) / (0.02 mL) = 2.43E+08 TU/mL; viral titre at 1 : 729 dilution = (13.3% × 40,000 cells × 729) / (0.02 mL) = 1.94 E+08 TU/mL. The average viral transduction titre detected with R177.B8 was calculated to be 2.19E+08 TU/mL. Similarly, the viral transduction titres detected by the R177.D13 and R177.G12 antibodies were calculated to be 2.09E+08 TU/mL and 1.97E+08 TU/mL, respectively. This indicates that the selected antibodies can be used for the detection of lentiviral transduction titres.

### Example 9. Detection of CAR-T cells by antibodies

According to the same procedure as described in Example 8, some of the antibodies were labelled with iFluor 647 (AAT Bioquest, CAT#: 1031).

According to the same procedure as described in Example 8, some of the antibodies were labelled with FITC.

Furthermore, some of the antibodies were labelled with PE. The antibody reduced by TECP (Thermo, CAT#: 20490) was added to SMCC (Thermo, CAT#: 22322)-activated phycoerythrin (PE) at a mass ratio of 0.5 : 1, followed by overnight reaction at 4°C. After the reaction was completed, the reaction solution was placed in an ultrafiltration tube and centrifuged at 12,000 × g for 10 min at 4°C. After the centrifugation was completed, the liquid in the outer tube was discarded, and the inner tube was replenished with PBS. The centrifugation-PBS replenishment process was repeated three times to replace the antibody-PE reaction buffer with PBS, thereby completing the PE labelling of the antibody.

1 × 10⁶ T cells, including 40% CAR-positive T cells and 60% CAR-negative T cells (in which the scFv on CAR contained a (G₄S)₃ linker), were washed once with 500 µl of FACS buffer (PBS + 1% BSA) and centrifuged at 500 × g for 5 minutes. The supernatant was then discarded. The cells were resuspended in 200 µl of FACS buffer, and 2 µg of iFluor 647-labeled R177.G5, PE-labelled R177.I8, FITC-labelled Protein L (Acro, CAT#: RPL-PF141), or iFluor 647-labeled anti-Fab antibody (Jackson, CAT#: 109-605-006) was added, followed by incubation at room temperature for 20 min. The cells were washed once with 500 µl of FACS buffer and centrifuged at 500 × g for 5 minutes. The supernatant was then discarded. The cells were resuspended in 200 µl of FACS buffer. The proportion of positive cells (i.e., the percentage of fluorescent cells relative to total cells) was detected by flow cytometry.

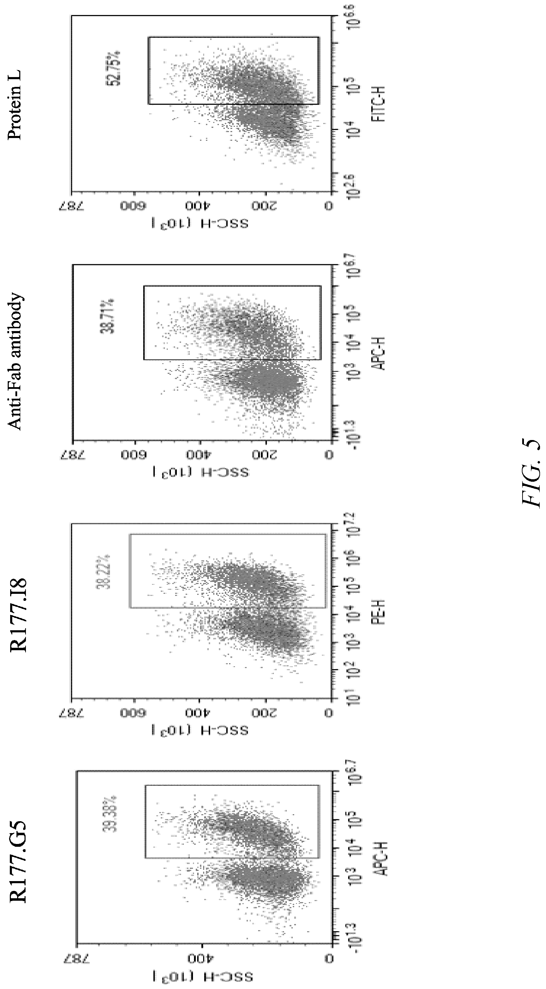

As shown in the results of FIG. 5, the antibodies of the present application, the anti-Fab antibody, and Protein L were all capable of characterizing CAR-T cells. However, the positive rate of CAR-T cells detected by Protein L was 52.75%, which differed greatly from 40%, indicating low accuracy. In addition, Protein L exhibited poor population resolution, high background signal, and high non-specific adsorption. The positive rates detected by the anti-Fab antibody, R177.G5, and R177.I8 were 38.71%, 39.26%, and 38.22%, respectively, all of which were close to 40%. It can be seen that these antibodies are all capable of accurately detecting the positive rate of CAR-T cells. In contrast, the cell population resolution after staining with the antibodies of the present application was significantly better than that with the anti-Fab antibody, which facilitates the identification of positive cell populations.

In summary, the GS linker antibodies of the present application are superior to Protein L and anti-Fab antibodies in the characterization of CAR-T cells.

### Example 10. Purification of GS linker-containing scFv by antibodies

The antibodies of the present disclosure were used to purify an scFv containing a (G₄S)₃ linker. The linker-containing scFv was prepared based on the sequence of CD19-targeting FMC63, which comprises, from the N-terminus to the C-terminus, a light chain variable region, a linker, and a heavy chain variable region. The amino acid sequences of the light chain variable region and heavy chain variable region are set forth in SEQ ID NO: 80 and SEQ ID NO: 81, respectively.

The antibody R177.B8 of the present disclosure was conjugated to the purification medium CNBr Focurose 4FF (Huiyan Bio, CAT#: HQ030301025M) at different mass-to-volume ratios (antibody : medium = 10 : 1, 15 : 1, 20 : 1) to obtain antibody-conjugated purification media. 100 µL of the antibody-coupled purification medium was added to a gravity column. Once the liquid had flowed through, 1 mL of *E. coli* lysate containing the scFv with the (G₄S)₃ linker was applied to the column. Once the liquid had flowed through, the medium was washed twice with 1 mL of PBS each time. Elution was then performed using 0.1 M glycine (pH 3.0). The eluate was collected and neutralized with 1 M Tris-HCl (pH 9.0). Protein concentration was quantified using a BCA kit (Thermo, CAT#: 23225) according to the operation method described in the instructions.

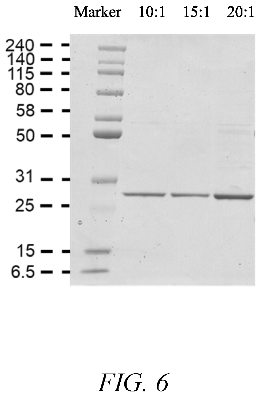

The scFv containing the G₄S)₃ linker purified at different conjugation ratios (antibody : medium = 10 : 1, 15 : 1, 20 : 1) was analysed by SDS-PAGE using a 4-20% precast gel (GenScript, CAT#: M00656). The obtained gel was stained using an eStain^{®} L1 protein stainer (GenScript, CaT#: L00657C), and the molecular size and purity were estimated by comparing the stained bands with a protein marker (GenScript, CaT#: M00624). As shown in FIG. 6, the molecular weights of the antibodies purified at different conjugation ratios were all about 28kd, which were consistent with the expected molecular weight, and the protein purity was ≥ 95%. This indicates that the selected antibodies can be used for the purification of scFv containing a GS linker. Lane 1: protein marker; Lane 2: scFv containing the (G₄S)₃ linker purified by the conjugated medium prepared at a ratio of antibody : medium of 10 : 1; Lane 3: scFv containing the (G₄S)₃ linker purified by the conjugated medium prepared at a ratio of antibody : medium of 15 : 1; and Lane 4: scFv containing the (G₄S)₃ linker purified by the conjugated medium prepared at a ratio of antibody : medium of 20 : 1.

**Table 3. Some of the amino acid and nucleotide sequences mentioned herein**

| SEQ ID NO | Description | Sequence |
|---|---|---|
| 48 | Heavy chain variable region of R177.B8 | CAGTCGCTGGAGGAGTCCGGGGGTCGCCTGGTAACGCC TGGAGGATCCCTGACACTCACCTGTACAGTCTCTGGAAT CGACCTCAGTAACTATGCAATAATGTGGGTCCGCCAGG CTCCAGGGAAGGGGCTGGAATGGATCGGAGTCATTTAT GCTAATGGTGACCCATACTGCGCGAGCTGGGCGAAAGG CCGATTCACCATCTCCAAAACCTCGTCGACCACGGTGGA TCTGAAAATGACCAGTCTGACAACCGAGGACACGGCCA CCTATTTCTGTGCCAGAGGGGGCTTTTGGGGCCCAGGCA CCCTGGTCACTGTCTCCTCA |
| 49 | Heavy chain variable | CAGTCGCTGGAGGAGTCCGGGGGTCGCCTGGTCACGCC TGGAGGATCCCTGACACTCACCTGCACAGCCTCTGGAAT |
| | region of R177.B11 | CGACCTCAGGAATTACGACATGTACTGGGCCCGCCAGG CTCTAGGGAAGGGGCTGGAATGGGTCGGAGTCATTTAT GTTAATGGAAACACACACTACGCGAGCTGGGCGAAAGG CCGATTCACCATCTCCAGAACCTCGTCGACCACGGTGGA TCTGAAAATGACCAGTCTGACAACCGAGGACACGGCCA CCTATTTTTGTGTCAGAGGGCCCTTCTGGGGCCCAGGCA CCCTGGTCACCGTCTCCTCA |
| 50 | Heavy chain variable region of R177.D13 | CAGTCGCTGGAGGAGTCCGGGGGTCGCCTGATAACGCC TGGAGGATCCCTGACACTCACCTGCACAGTCTCTGGAAT CGACCTCAGTAATCATGCAATAATGTGGGTCCGCCAGG CTCCAGGGAAGGGGCTGGAATGGATCGGAGTCATTTAT AGTAATGGAAATCCATACTGCGCGAGGTGGGTGAAAGG CCGATTCACCATCTCCAAAACCTCGTCGACCACGGTGGA TCTGAAAATGACCAGTCTGACAACCGAGGACACGGCCA CCTATTTTTGTGCCAGAGGGGGCTTTTGGGGCCCAGGCA CCCTGGTCACCGTCTCCTCA |
| 51 | Heavy chain variable region of R177.E4 | CAGTCGCTGGAGGAGTCCGGGGGTCGCCTGGTCACGCC TGGAGGATCCCTGACACTCACCTGCACAGCCTCTGGAAT CGACCTCAAGAACTATGACATGTATTGGGCCCGCCAGG CTCCAGGGAAGGGGCTGGATTGGATCGGAGTCAGTTAT AAAAATGGTCGCGCACACTACGCGAGCTGGGCGAAAGG CCGATTCACCATCTCCAAGACCTCGACCACGGTGGATCT GAAAATGACCAGTCTGACAACCGAGGACACGGCCACCT ATTTTTGTGTCAGGGGGGCCCTTGTGCGGCCCAGGTACCC TGGTCACCGTCTCCTCA |
| 52 | Heavy chain variable region of R177.G3 | CAGTCGCTGGAGGAGTCCGGGGGTCGCCTGGTCACGCC TGGAGGATCCCTGACACTCACCTGCACAGCCTCTGGAAT CGACCTCAATAACTACGACATGTACTGGGCCCGCCAGG CTCCAGGGAAGGGGCTGGAGTGGATCGGATGCATTTAT ACTGGTAGTGGTGGCAAACGCTACGCGAACTGGGCGAA AGGCCGATTCACCTTCTCCAGAACCTCGACCACGGTGGA TCTGAAAATGACCAGTCTGACAACCGAGGACACGGCCA CCTATTTTTGTGTCAGAGGGCCCTTCTGGGGCCCAGGCA CCCTGGTCACCGTCTCCTCA |
| 53 | Heavy chain variable region of R177.G5 | CAGTCGCTGGAGGAGTCCGGGGGTCGCCTGGTCACGCC TGGAGGATCCCTGACACTCACCTGCACAGCCTCTGGAAT CGACCTCAACAACTACGACATGTACTGGGCCCGCCAGG CTCCAGGGAAGGGGCTGGATTGGATCGGAGTCAGTTAT GCTAGTGGCAGGACATATTCAGTGAGGTGGGCGAAAGG CCGATTCACCATCTCCAAAACCTCGTCGACCACGGTGGA TCTGAAAATGACCAGTCTGACAACCGAGGACACGGCCA CCTATTTTTGTGTCAGAGGGCCCTTGTGGGGCCCAGGCA CCCTGGTCACCGTCTCCTCA |
| 54 | Heavy chain variable region of R177.G12 | CAGTCGGTGGAGGAGTCCGGGGGTCGCCTGGTCACGCC TGGGACACCCCTGACACTCACCTGCACCGTCTCTGGATT CTCCCTCAGTACCAATGCAATGACCTGGGTCCGCCAGGC TCCAGGGGAGGGGCTGGAATGGATCGGAACCATTACTA TTAGTGGTAATAAATACTACGCGAGCTGGGCGAAAGGC CGATTCACCATCTCCAGAACCTCGACCACGGTGGATCTG AAAATGACCAGTCTGACAGCCGCGGACACGGCCACCTA TTTCTGTGCCAGAGGAGTTGTTCAGAGCCTTGTCTTGTG GGGCCAAGGCACCCTGGTCAGCGTCTCCTCA |
| 55 | Heavy chain variable region of R177.I8 | CAGTCGCTGGAGGAGTCCGGGGGTCGCCTGGTCACGCC TGGAGGATTTCTGACACTCACCTGTATAGCCTCTGGAAT CGACCTCAGGAATTACGACATGTATTGGGCCCGCCAGG CTCTAGGGAAGGGGCTGGAATGGGTCGGAGTCATTTAT AAAAATGGAAACGCACACTCCGCGAGCTGGGCGAGAGG CCGATTCACCATCTCCAGAACCTCGTCGACCACGGTGGA TCTGAAAATGACCAGTCTGACAACCGAGGACACGGCCA CCTATTTTTGTGTCAGAGGGCCCTTCTGGGGCCCAGGCA CCCTGGTCACCGTCTCCTCA |
| 56 | Light chain variable region of R177.B8 | CAAGTGCTGACCCAGACTCCATCCCCTGTGTCTGCAGCT GTGGGAGGCACAGTCACCATCAATTGCCAGTCCAGTCA GAGTATTTATAATAAGAACGACTTAGTCTGGCTTCAGCA GAAACCAGGGCAGCCTCCCAAGCGCCTGATCTATGGTA TATCCACTCTGGATTCTGGGGTCTCATCGCGGTTCAAGG GCAGTGGATCTGGGACACAGTTCACTCTCACCATCAGCG GCGTGCAGTGTGACGATGCTGCCACTTACTACTG TCTAGGCGGCTTTAGTTGTAGTAGTGGTGATTGTGGTGC TTTCGGCGGAGGGACCGAGGTGGTGGTCAGA |
| 57 | Light chain variable region of R177.B11 | GCCCAAGTGCTGACCCAGACTCCATCCCCTGTGTCTGCA GCTGTGGGAGGCACAGTCACCATCAATTGCCAGGCCAG TCAGAGTGTTTGGAAGAACAAGGACTTAGTCTGGTATC AGCAGAAAGCAGGGCAGCCTCCTAAACGCCTGATCTAT GATGCATCCACTCTGTCATCTGGGGTCTCATCGCGGTTC AAAGGCAGTGGATCTGGGACACAGTTCAGTCTCACCAT GACCGACGTGCAGTGTGACGATGCTGCCACTTACTATTG TCTAGGCGGATTTAGTTGTAGTCGTGGTGATTGTGGTGC TTTCGGCGGAGGGACCGAGGTGGTGGTCAA |
| 58 | Light chain variable region of R177.D13 | GCCCAAGTGCTGACCCAGACTCTATCCCCTGTGTCTGCA GCTGTGGGAGGCACAGTCACCATCAATTGCAAGACCAG TCAGAGTATCTATAATAACAACGACTTAGTCTGGCTTCA GCAGAAACCAGGGCAGCCTCCCAAGCGCCTGATCTATG GTGTATCCACTCTGGATTCTGGGGTCTCATCGCGGTTCA AGGGCAGTGGATCTGGGACACAGTTCACTCTCACCATC AGCGGCATGCAGTGTGAAGATGCTGCCACTTACTACTGT |
| | | CTAGGCGGCTTTCGTTGTAGTAGTGGTGATTGTGGTGCT TCCGGCGGAGGGACCGAGGTGGTGGTCAGA |
| 59 | Light chain variable region of R177.E4 | GCCCAAGTGCTGACCCAGACTCCATCCCCTGTGTCTGCA GCTGTGGGAGGCACAGTCACCATCAATTGCCAGGCCAG TCAGAGTGTTTGGAATAACAACGACTTAGTCTGGTATCA GCAGAAACCAGGGCAGCGTCCCAAGCGCCTGATCTATG ATGCATCCACTCTGTCATCTGGGGTCTCATCGCGGTTCA AAGGCAGTGGATCTGGGACACAGTTCACTCTCACCATC AGCGGCGTGCAGTGTGACGATGCGGCCACTTACTACTGT CTAGGCGGATTTAGTTGTAGTACTGGTGATTGTGGTGCT TTCGGCGGAGGGACCGAGGTGGTGGTCAAA |
| 60 | Light chain variable region of R177.G3 | GCCCAAGTGCTGACCCAGACTCCATCCCCTGTGTCTGCA GCTGTGGGAGGCACAGTCACCATCATTTGCCAGGCCAG TCAGAGTGTTTGGAATAACAACGACTTAGTCTGGTATCA GCAGAAAGCAGGGCAGCGTCCCAAGCGCCTGATCTATG ATGCATCCATTCTGTCATCTGGGGTCTCATCGCGGTTCA AAGGCAGTGGATCTGGGACACAGTTCAGTCTCACCGTG AGCGACGTGCAGTGTGACGATGCTGCCACTTACTATTGT CTAGGCGGATTTAGTTGTCGTAGTGGTGATTGTGGTGCT TTCGGCGGAGGGACCGAGGTGGTGGTCAAA |
| 61 | Light chain variable region of R177.G5 | GCCCAAGTGCTGACCCAGACTCCATCCCCTGTGTCTGCA GCTGTGGGAGGCACAGTCACCATCAATTGCCAGGCCAG TCAGACTGTTTGGAAGAACAACGACTTAGTCTGGTATCA GCAGAAACCAGGGCAGCGTCCCAAGCGCCTGATCTATG ATGCATCCACTCTGTCATCTGGGGTCTCATCGCGATTCA AAGGCAGTGGATCTGGGACACAGTTCAGTCTCACCATG AGCGACGTGCAGTGTGACGATGCTGCCACTTACTACTGT CTAGGCGGATTTAGTTGTAGTAGTGGTGATTGTGGTGCT TTCGGCGGAGGGACCGAGGTGGTGGTCAAA |
| 62 | Light chain variable region of R177.G12 | GCCGTCGTAATGACCCAGACTCCAGCCTCCGTGTCTGCA GCTGTGGGAGGCACAGTCACCATCAGTTGCCGGTCCAG TCAGAATGTGTATAATAATAACGGCTTAGGCTGGTATCA GCAGAAACCAGGGCAGCCTCCCAAGCTCCTGATCTATG ATGCAGCCGATCTGGCATCTGGGGTCCCATCGCGGTTTA AAGGCAGTGGATCTGGGACACAGTTCACTCTCACCATC AGCGACCTGGAGTGTGACGATGCTGCCACTTACTACTGT GCAGGCGGTTATAGTAGTGGTAGTATTGATAATACTTTC GGCGGAGGGACCGAGGTGGTGGTCAAA |
| 63 | Light chain variable region of R177.I8 | GCCCAAGTGCTGACCCAGACTCCATCCCCTGTGTCTGCA GCTGTGGGAGGCACAGTCACCATCAATTGCCAGGCCAG TCAGAGTGTTTGGAATAACAACGACTTAGTCTGGTATCA GCAGAAACCAGGGCAGCCTCCTAAACGCCTGATCTATG ATGCATCCACTCTGTCATCTGGGGTCTCATCGCGGTTCA |
| | | GAGGCAGTGGATCTGGACACAGTTCAGTCTCACCATG ACCGACGTGCAGTGTGACGATGCTGCCACTTACTATTGT CTAGGCGGATTTAGTTGTAGTCGTGGTGATTGTGGTTCT TTCGGCGGAGGGACCGAGGTGGTGGTCAAA |
| 64 | Heavy chain constant region | GQPKAPSVFPLAPCCGDTPSSTVTLGCLVKGYLPEPVTVT WNSGTLTNGVRTFPSVRQSSGLYSLSSVVSVTSSSQPVTCN VAHPATNTKVDKTVAPSTCSKPTCPPPELLGGPSVFIFPPKP KDTLMISRTPEVTCVVVDVSQDDPEVQFTWYINNEQVRTA RPPLREQQFNSTIRVVSTLPIAHQDWLRGKEFKCKVHNKA LPAPIEKTISKARGQPLEPKVYTMGPPREELSSRSVSLTCMI NGFYPSDISVEWEKNGKAEDNYKTTPAVLDSDGSYFLYSK LSVPTSEWQRGDVFTCSVMHEALHNHYTQKSISRSPGK |
| 65 | Heavy chain constant region | GGGCAACCTAAGGCTCCATCAGTCTTCCCACTGGCCCCC TGCTGCGGGGACACACCCAGCTCCACGGTGACCCTGGG CTGCCTGGTCAAAGGCTACCTCCCGGAGCCAGTGACCGT GACCTGGAACTCGGGCACCCTCACCAATGGGGTACGCA CCTTCCCGTCCGTCCGGCAGTCCTCAGGCCTCTACTCGC TGAGCAGCGTGGTGAGCGTGACCTCAAGCAGCCAGCCC GTCACCTGCAACGTGGCCCACCCAGCCACCAACACCAA AGTGGACAAGACCGTTGCGCCCTCGACATGCAGCAAGC CCACGTGCCCACCCCCTGAACTCCTGGGGGGACCGTCTG TCTTCATCTTCCCCCCAAAACCCAAGGACACCCTCATGA TCTCACGCACCCCCGAGGTCACATGCGTGGTGGTGGAC GTGAGCCAGGATGACCCCGAGGTGCAGTTCACATGGTA CATAAACAACGAGCAGGTGCGCACCGCCCGGCCGCCGC TACGGGAGCAGCAGTTCAACAGCACGATCCGCGTGGTC AGCACCCTCCCCATCGCGCACCAGGACTGGCTGAGGGG CAAGGAGTTCAAGTGCAAAGTCCACAACAAGGCACTCC CGGCCCCCATCGAGAAAACCATCTCCAAAGCCAGAGGG CAGCCCCTGGAGCCGAAGGTCTACACCATGGGCCCTCC CCGGGAGGAGCTGAGCAGCAGGTCGGTCAGCCTGACCT GCATGATCAACGGCTTCTACCCTTCCGACATCTCGGTGG AGTGGGAGAAGAACGGGAAGGCAGAGGACAACTACAA GACCACGCCGGCCGTGCTGGACAGCGACGGCTCCTACT TCCTCTACAGCAAGCTCTCAGTGCCCACGAGTGAGTGGC AGCGGGGCGACGTCTTCACCTGCTCCGTGATGCACGAG GCCTTGCACAACCACTACACGCAGAAGTCCATCTCCCGC TCTCCGGGTAAA |
| 66 | Light chain constant region | GDPVAPTVLIFPPAADQVATGTVTIVCVANKYFPDVTVTW EVDGTTQTTGIENSKTPQNSADCTYNLSSTLTLTSTQYNSH KEYTCKVTQGTTSVVQSFNRGDC |
| 67 | Light chain constant region | GGTGATCCAGTTGCACCTACTGTCCTCATCTTCCCACCA GCTGCTGATCAGGTGGCAACTGGAACAGTCACCATCGT GTGTGTGGCGAATAAATACTTTCCCGATGTCACCGTCAC CTGGGAGGTGGATGGCACCACCCAAACAACTGGCATCG AGAACAGTAAAACACCGCAGAATTCTGCAGATTGTACC TACAACCTCAGCAGCACTCTGACACTGACCAGCACACA GTACAACAGCCACAAAGAGTACACCTGCAAGGTGACCC AGGGCACGACCTCAGTCGTCCAGAGCTTCAATAGGGGT GACTGT |
| 74 | Heavy chain variable region of brolucizumab | MGWSCIILFLVATATGVHSEIVMTQSPSTLSASVGDRVIITC QASEIIHSWLAWYQQKPGKAPKLLIYLASTLASGVPSRFSG SGSGAEFTLTISSLQPDDFATYYCQNVYLASTNGANFGQG TKLTVL |
| 75 | Light chain variable region of brolucizumab | EVQLVESGGGLVQPGGSLRLSCTASGFSLTDYYYMTWVR QAPGKGLEWVGFIDPDDDPYYATWAKGRFTISRDNSKNTL YLQMNSLRAEDTAVYYCAGGDHNSGWGLDIWGQGTLVT VSSHHHHHH |
| 76 | Heavy chain variable region of rituximab | |
| 77 | Light chain variable region of rituximab | |
| 78 | Heavy chain variable region of muromonab | |
| 79 | Light chain variable region of muromonab | QIVLTQSPAIMSASPGEKVTMTCSASSSVSYMNWYQQKSG TSPKRWIYDTSKLASGVPAHFRGSGSGTSYSLTISGMEAED AATYYCQQWSSNPFTFGSGTKLEINHHHHHH |
| 80 | Light chain variable region of FMC63 | WSHPQFEKDIQMTQTTSSLSASLGDRVTISCRASQDISKYL NWYQQKPDGTVKLLIYHTSRLHSGVPSRFSGSGSGTDYSL TISNLEQEDIATYFCQQGNTLPYTFGGGTKLEIT |
| 81 | Heavy chain variable | EVKLQESGPGLVAPSQSLSVTCTVSGVSLPDYGVSWIRQPP RKGLEWLGVIWGSETTYYNSALKSRLTIIKDNSKSQVFLK |
| | region of FMC63 | MNSLQTDDTAIYYCAKHYYYGGSYAMDYWGQGTSVTVS SHHHHHH |

Although the present application has been described in conjunction with one or more embodiments, it should be understood that the present application is not limited to these embodiments. The description in the present application is intended to cover all variations and equivalents, all of which are included within the spirit and scope of the appended claims. All documents cited herein are incorporated by reference in their entireties.

## Claims

1. An isolated monoclonal antibody or an antigen-binding portion thereof that is capable of specifically binding to a GS linker, **characterised in that** the antibody or the antigen-binding portion thereof comprises: i) a heavy chain variable region comprising VH CDR1, VH CDR2, and VH CDR3, and ii) a light chain variable region comprising VL CDR1, VL CDR2, and VL CDR3, wherein VH CDR1, VH CDR2, VH CDR3, VL CDR1, VL CDR2, and VL CDR3 comprise amino acid sequences set forth in:
(1) NYAIM (SEQ ID NO: 1), VIYANGDPYCASWAKG (SEQ ID NO: 5), GGF, QSSQSIYNKNDLV (SEQ ID NO: 14), GISTLDS (SEQ ID NO: 20), and LGGFSCSSGDCGA (SEQ ID NO: 25), respectively;
(2) NYDMY (SEQ ID NO: 2), VSYKNGRAHYASWAKG (SEQ ID NO: 8), GPL, QASQSVWNNNDLV (SEQ ID NO: 17), DASTLSS (SEQ ID NO: 21), and LGGFSCSTGDCGA (SEQ ID NO: 28), respectively;
(3) NYDMY (SEQ ID NO: 2), VSYASGRTYSVRWAKG (SEQ ID NO: 10), GPL, QASQTVWKNNDLV (SEQ ID NO: 18), DASTLSS (SEQ ID NO: 21), and LGGFSCSSGDCGA (SEQ ID NO: 25), respectively;
(4) NYDMY (SEQ ID NO: 2), VIYKNGNAHSASWARG (SEQ ID NO: 12), GPF, QASQSVWNNNDLV (SEQ ID NO: 17), DASTLSS (SEQ ID NO: 21), and LGGFSCSRGDCGS (SEQ ID NO: 31), respectively;
(5) NHAIM (SEQ ID NO: 3), VIYSNGNPYCARWVKG (SEQ ID NO: 7), GGF, KTSQSIYNNNDLV (SEQ ID NO: 16), GVSTLDS (SEQ ID NO: 22), and LGGFRCSSGDCGA (SEQ ID NO: 27), respectively;
(6) NYDMY (SEQ ID NO: 2), VIYVNGNTHYASWAKG (SEQ ID NO: 6), GPF, QASQSVWKNKDLV (SEQ ID NO: 15), DASTLSS (SEQ ID NO: 21), and LGGFSCSRGDCGA (SEQ ID NO: 26), respectively;
(7) NYDMY (SEQ ID NO: 2), CIYTGSGGKRYANWAKG (SEQ ID NO: 9), GPF, QASQSVWNNNDLV (SEQ ID NO: 17), DASILSS (SEQ ID NO: 23), and LGGFSCRSGDCGA (SEQ ID NO: 29), respectively; or
or (8) TNAMT (SEQ ID NO: 4), TITISGNKYYASWAKG (SEQ ID NO: 11), GVVQSLVL (SEQ ID NO: 13), RSSQNVYNNNGLG (SEQ ID NO: 19), DAADLAS (SEQ ID NO: 24), and AGGYSSGSIDNT (SEQ ID NO: 30), respectively; or amino acid sequences having 1 to 3 amino acid substitutions in each CDR as compared to the above amino acid sequences.

2. The antibody or the antigen-binding portion thereof according to claim 1, **characterised in that** the heavy chain variable region comprises an amino acid sequence having at least 80%, 85%, 90%, 91%, 92%, 93%, 94%, 95%, 96%, 97%, 98%, 99%, or 100% sequence identity to SEQ ID NO: 32, 35, 37, 39, 34, 33, 36, or 38.

3. The antibody or the antigen-binding portion thereof according to claim 1, **characterised in that** the light chain variable region comprises an amino acid sequence having at least 80%, 85%, 90%, 91%, 92%, 93%, 94%, 95%, 96%, 97%, 98%, 99%, or 100% sequence identity to SEQ ID NO: 40, 43, 45, 47, 42, 41, 44, or 46.

4. The antibody or the antigen-binding portion thereof according to claim 1, **characterised in that** the heavy chain variable region and the light chain variable region comprise amino acid sequences having at least 80%, 85%, 90%, 91%, 92%, 93%, 94%, 95%, 96%, 97%, 98%, 99%, or 100% sequence identity to: (1) SEQ ID NOs: 32 and 40, respectively; (2) SEQ ID NOs: 35 and 43, respectively; (3) SEQ ID NOs: 37 and 45, respectively; (4) SEQ ID NOs: 39 and 47, respectively; (5) SEQ ID NOs: 34 and 42, respectively; (6) SEQ ID NOs: 33 and 41, respectively; (7) SEQ ID NOs: 36 and 44, respectively; or (8) SEQ ID NOs: 38 and 46, respectively.

5. The antibody or the antigen-binding portion thereof according to claim 1, **characterised in that** the antibody or the antigen-binding portion further comprises a heavy chain constant region and/or a light chain constant region.

6. The antibody or the antigen-binding portion thereof according to claim 1, **characterised in that** the antibody or the antigen-binding portion further comprises a detectable substance.

7. The antibody or the antigen-binding portion thereof according to claim 6, **characterised in that** the detectable substance is a fluorescent label, biotin, or a chemiluminescent label.

8. The antibody or the antigen-binding portion thereof according to claim 1, **characterised in that** the antibody or the antigen-binding portion thereof is a rabbit antibody, a chimeric antibody, or a humanized antibody.

9. An isolated bispecific molecule, comprising the antibody or the antigen-binding portion thereof according to any one of claims 1 to 8.

10. The bispecific molecule according to claim 9, **characterised in that** the bispecific molecule further comprises an antibody or an antigen-binding portion thereof that targets a disease-associated antigen.

11. A chimeric antigen receptor, comprising:
a) an extracellular domain comprising the antibody according to any one of claims 1 to 4 and 8 that is in the form of a single-chain antibody,
b) a transmembrane domain, and
c) an intracellular signalling domain.

12. A nucleic acid molecule, encoding the antibody or the antigen-binding portion thereof according to any one of claims 1 to 8, the bispecific molecule according to claim 9 or 10, or the chimeric antigen receptor according to claim 11.

13. The nucleic acid molecule according to claim 12, **characterised in that** the nucleic acid molecule comprises a nucleotide sequence set forth in any one of SEQ ID NOs: 48-63.

14. An expression vector, comprising the nucleic acid molecule according to claim 12 or 13.

15. A host cell, comprising the expression vector according to claim 14, or having the nucleic acid molecule according to claim 12 or 13 integrated into the genome thereof.

16. A composition, comprising the antibody or the antigen-binding portion thereof according to any one of claims 1 to 8, the bispecific molecule according to claim 9 or 10, the chimeric antigen receptor according to claim 11, the nucleic acid molecule according to claim 12 or 13, the expression vector according to claim 14, or the host cell according to claim 15.

17. The composition according to claim 16, **characterised in that** the antibody or the antigen-binding portion thereof comprises a chemiluminescent label, and the composition further comprises a reagent that causes the chemiluminescent label to emit light.

18. The composition according to claim 16, **characterised in that** the antibody or the antigen-binding portion thereof comprises an Fc region, and the composition further comprises an antibody that specifically binds to the Fc region and that comprises a detectable substance.

19. The composition according to claim 18, **characterised in that** i) the detectable substance contained in or conjugated to the antibody that specifically binds to the Fc region is a fluorescent label; or ii) the detectable substance contained in the antibody that specifically binds to the Fc region is a chemiluminescent label, and the composition further comprises a reagent that causes the chemiluminescent label to emit light.

20. The composition according to claim 16, **characterised in that** the composition comprises:
i) the bispecific molecule according to claim 9 or 10; and
ii) a T cell expressing a chimeric antigen receptor, wherein the chimeric antigen receptor comprises:
a) an extracellular domain comprising a non-antigen-binding single-chain antibody that comprises a GS linker,
b) a transmembrane domain, and
c) an intracellular signalling domain; or
i) a T cell expressing the chimeric antigen receptor according to claim 11, and
ii) an antibody or an antigen-binding portion thereof that is linked to a GS linker and that targets a disease-associated antigen.

21. A method for detecting a GS linker-containing molecule in a sample, comprising:
i) contacting the sample with the antibody or the antigen-binding portion thereof according to any one of claims 1 to 8, and
ii) detecting the presence of the antibody or the antigen-binding portion thereof in the sample,
wherein the presence of the antibody or the antigen-binding portion thereof in the sample indicates the presence of a GS linker-containing molecule in the sample.

22. A method for purifying a GS linker-containing molecule from a sample, comprising:
i) contacting the sample with a solid support conjugated to the antibody or the antigen-binding portion thereof according to any one of claims 1 to 8,
ii) separating from the sample the molecule bound to the solid support in step i) in the sample, and
iii) eluting from the solid support the molecule bound to the solid support,
wherein the solid support includes magnetic beads, resin, and agarose beads; and the molecule bound to the solid support is a GS linker-containing molecule.

23. The method according to claim 21 or 22, **characterised in that** the GS linker is (G₂S)₂ (SEQ ID NO: 69), (G₂S)₄ (SEQ ID NO: 70), (G₃S)₃ (SEQ ID NO: 71), (G₄S)₂ (SEQ ID NO: 73), or (G₄S)₃ (SEQ ID NO: 68).

24. The method according to claim 21 or 22, **characterised in that** the GS linker-containing molecule is a GS linker-containing antibody or a GS linker-containing cell.

25. The method according to claim 24, **characterised in that** the sample comprises cells treated with a GS linker-containing antibody, or cells into which a vector has been introduced, wherein the vector comprises a sequence for expressing a GS linker-containing chimeric antigen receptor.

26. The method according to claim 21 or 22, **characterised in that** the GS linker-containing molecule in the sample is quantified on the basis of an amount of the antibody or the antigen-binding portion thereof in the sample.

27. Use of the composition according to claim 20 in the preparation of a drug for treating a disease, **characterised in that** the disease is associated with a disease-associated antigen targeted by a bispecific molecule in a composition, or by an antibody or an antigen-binding portion thereof that comprises a GS linker and that targets the disease-associated antigen.
